# EUROPEAN PATENT SPECIFICATION

(11) **EP 1 487 879 B1**
(45) Date of publication and mention of the grant of the patent: **26.12.2012**
(21) Application number: 03743416.4
(22) Date of filing: 03.03.2003
(51) Int. Cl.: C07K 16/46, A61K 47/48, A61K 51/10, A61P 35/00, A61P 37/00, A61P 31/00, G01N 33/577

(54) **BISPECIFIC ANTIBODY POINT MUTATIONS FOR ENHANCING RATE OF CLEARANCE**
PUNKTMUTATIONEN BEI BISPEZIFISCHEN ANTIKÖRPERN ZUR VERBESSERUNG DER CLEARANCE-RATE
MUTATIONS PONCTUELLES DANS UN ANTICORPS BISPECIFIQUE, PERMETTANT D'AUGMENTER LE TAUX DE CLAIRANCE

(30) Priority: 01.03.2002 US 361037 P
(43) Date of publication of application: 22.12.2004
(73) Proprietor: Immunomedics, Inc., Morris Plains, NJ 07950 (US)
(72) Inventor: QU, Zhengxing, Morris Plains, NJ 07950 (US); HANSEN, Hans, Morris Plains, NJ 07950 (US); GOLDENBERG, David, M., Morris Plains, NJ 07950 (US)
(74) Representative: Bohmann, Armin K.
(86) International application number: PCT/GB2003/000871
(87) International publication number: WO 2003/074569

(56) References cited:
- WO-A-93/22332
- WO-A-99/66951
- US-A1- 2005 100 543
- KARACAY H ET AL: "Experimental pretargeting studies of cancer with a humanized anti-CEA * Murine anti-(In-DTPA) Bispecific antibody construct and a 99mTc/188Re-labeled Peptide" BIOCONJUGATE CHEMISTRY, AMERICAN CHEMICAL SOCIETY, WASHINGTON, US, vol. 11, 2000, pages 842-854, XP002230983 ISSN: 1043-1802
- GESTIN J F ET AL: "Two-step targeting of xenografted colon carcinoma using a bispecific antibody and 188Re-labeled bivalent hapten: biodistribution and dosimetry studies" JOURNAL OF NUCLEAR MEDICINE, SOCIETY OF NUCLEAR MEDICINE. NEW YORK, US, vol. 42, no. 1, January 2001 (2001-01), pages 146-153, XP002178931 ISSN: 0161-5505
- HORNICK JASON L ET AL: "Single amino acid substitution in the Fc region of chimeric TNT-3 antibody accelerates clearance and improves immunoscintigraphy of solid tumors" JOURNAL OF NUCLEAR MEDICINE, vol. 41, no. 2, February 2000 (2000-02), pages 355-362, XP002261694 ISSN: 0161-5505 cited in the application

## Description

### Background of the Invention

### Field of the Invention

The present invention relates to a mutant bispecific antibody (bsAb) which clears from a patient's body faster than the corresponding parent bsAb. In particular, the invention relates to a mutant bsAb, containing a human hinge constant region from IgG, two scFvs and two Fvs, wherein the hinge constant region contains one or more amino acid mutations in the C_{H}2, a product comprising such bsAb, a kit comprising such bsAb and the use of such bsAb.

### Related Art

The detection of a target site benefits from a high signal-to-background ratio of a detection agent. Therapy benefits from as high an absolute accretion of therapeutic agent at the target site as possible, as well as a reasonably long duration of uptake and binding. In order to improve the targeting ratio and amount of agent delivered to a target site, the use of targeting vectors comprising diagnostic or therapeutic agents conjugated to a targeting moiety for preferential localization has long been known.

Examples of targeting vectors include diagnostic or therapeutic agent conjugates of targeting moieties such as antibody or antibody fragments, cell- or tissue-specific peptides, and hormones and other receptor-binding molecules. For example, antibodies against different determinants associated with pathological and normal cells, as well as associated with pathogenic microorganisms, have been used for the detection and treatment of a wide variety of pathological conditions or lesions. In these methods, the targeting antibody is directly conjugated to an appropriate detecting or therapeutic agent as described, for example, in Hansen et al. , U.S. Pat. No. 3, 927,193 and Goldenberg, U.S. Pat. Nos. 4,331,647, 4,348,376, 4,361,544, 4,468,457, 4,444,744, 4,460,459, 4,460,561, 4,624,846 and 4,818,709.

One problem encountered in direct targeting methods, i.e., in methods wherein the diagnostic or therapeutic agent (the "active agent") is conjugated directly to the targeting moiety, is that a relatively small fraction of the conjugate actually binds to the target site, while the majority of conjugate remains in circulation and compromises in one way or another the function of the targeted conjugate. In the case of a diagnostic conjugate, for example, a radioinununoscintigraphic or magnetic resonance imaging conjugate, non-targeted conjugate which remains in circulation can increase background and decrease resolution. In the case of a therapeutic conjugate having a very toxic therapeutic agent, e.g., a radioisotope, drug or toxin, attached to a long-circulating targeting moiety, such as an antibody, circulating conjugate can result in unacceptable toxicity to the host, such as marrow toxicity or systemic side effects.

Pretargeting methods have been developed to increase the target:background ratios of the detection or therapeutic agents. Examples of pre-targeting and biotin/avidin approaches are described, for example, in Goodwin et al., U.S. Patent No. 4,863,713; Goodwin et al., J. Nucl. Med. 29:226, 1988; Hnatowich et al., J. Nucl. Med. 28:1294, 1987; Oehr et al., J. Nucl. Med. 29:728, 1988; Klibanov et al., J. Nucl. Med. 29:1951, 1988; Sinitsyn et al., J. Nucl. Med. 30:66, 1989; Kalofonos et al.. J. Nucl. Med. 31:1791, 1990; Schechter et al., Int. J. Cancer 48:167, 1991; Paganelli et al. , Cancer Res. 51:5960, 1991; Paganelli et al., Nucl. Med. Commun. 12:211, 1991; U.S. Patent No. 5,256,395; Stickney et al., Cancer Res. 51:6650, 1991; Yuan et al., Cancer Res. 51:3119, 1991; U.S. Patent No. 6,077,499; WO 01/097855; WO 1999/66951; WO 02/082041; WO 1997/34632; U.S. Patent No. 6,090,381; U.S. Patent No.6,472,511; U.S. Provisional Application No. 60/386,411; U.S. Provisional Application No. 60/345,641; U.S. Provisional Application No. 60/3328,835; U.S. Provisional Application No. 60/426,379, and U.S. Provisional Application No. 60/342.103.

In pretargeting methods, a primary targeting species (which is not bound to a diagnostic or therapeutic agent) is administered. The primary targeting species comprises a targeting moiety which binds to the target site and a binding moiety which is available for binding to a binding site on a targetable construct. Once sufficient accretion of the primary targeting species is achieved, a targetable construct is administered. The targetable construct comprises a binding site which recognizes the available binding site of the primary targeting species and a diagnostic or therapeutic agent.

Pretargeting is an approach which offers certain advantages over the use of direct targeting methods. For example, use of the pretargeting approach for the in vivo delivery of radionuclides to a target site for therapy, e.g., radioimmunotherapy, reduces the marrow toxicity caused by prolonged circulation of a radioimmunoconjugate. This is because the radioisotope is delivered as a rapidly clearing, low molecular weight chelate rather than directly conjugated to a primary targeting molecule, which is often a long-circulating species.

A problem encountered with pretargeting methods is that circulating primary targeting species (primary targeting species which is not bound to the target site) interferes with the binding of the targetable conjugate to targeting species that are bound to the target site (via the binding moiety on the primary targeting species). Thus, there is a need for methods of minimizing the amount of circulating primary targeting species.

Some attempts have been made to minimize the amount of circulating primary targeting species. One method for obtaining this goal is to prepare a primary targeting species with an accelerated rate of clearance from the body. For example, Ward et al. (U.S. Patent No. 6,165,745) has synthesized a mutant IgG 1 from murine and Hornick et al. The Journal of Nuclear Medicine 11 355-362 (2000) has synthesized a mutant chimeric TNT-3 antibody. These mutant antibodies differ from the mutant bsAb of the present invention. One difference is that the inventive mutant bsAb of the present invention is a bispecific antibody, whereas the antibodies of Hornick et al. and Ward et al. are monospecific antibodies. This difference is significant because a bispecific antibody has different properties than a monospecific antibody. Another difference between the present mutant bsAb and the murine antibody of Ward et al. is that the murine antibody of Ward et al. does not have an effector function. Therefore, the antibody of Ward et al. is not capable of fixing complement or effecting ADCC (antibody dependent cell cytotoxicity), as is the present mutant bsAb.

International patent application WO 99/66951 discloses the use of bi-specific antibodies for pre-targeting diagnosis and therapy.

International patent application WO 93/22332 discloses the recombinant production of immunoglobulin domains in prokaryotic cells.

### Summary of the Invention

The problem underlying the present invention is to provide a mutant bispecific antibody (bsAb) which clears from a patient's body faster than the corresponding parent bsAb.

This problem is solved by the subject matter of the independent claims. Preferred embodiments may be taken from the dependent claims.

More specifically, the problem is solved in a first aspect by a mutant bispecific antibody comprising at least
a human hinge-Fc constant region from IgG;
two scFvs that each specifically binds to a binding site on a bivalent targetable construct such that a one to one binding interaction is obtained between the mutant bispecific antibody and the targetable construct; and
two Fvs,
wherein said constant region contains one or more amino acid mutations in the C_{H}2 domain, wherein said hinge constant region contains a mutation in which isoleucine at position 253 is replaced with alanine, or an amino acid other than leucine, and wherein the amino acid replacement enhances blood clearance equal to, or greater than replacement with alanine.

In an embodiment the scFvs and the Fvs are CDR-grafted murine scFvs and Fvs.

In an embodiment the scFvs and the Fvs are humanized or human.

In an embodiment of the bsAb the Fvs are derived from hMN14-IgG, a humanized Class III, anti-CEA mAb (see U.S. Patent No. 5,874,540) the scFvs are 734scFv.

In an embodiment the mutant bispecific antibody comprises the Fv regions of hMN14-IgG and 734scFv. In an embodiment the bivalent targetable construct is conjugated to one or more drugs, toxins, radioisotopes, enzymes or labels.

In an embodiment the Fvs bind to an epitope on a target cell.

In an embodiment the Fvs bind to a tumor associated antigen.

In an embodiment the Fvs bind to CEA (carcinoembryonic antigen).

In an embodiment the mutant bispecific antibody incorporates the Fv of a class III anti-CEA antibody.

In an embodiment the mutant bispecific antibody incorporates the scFv of Mab 679.

The problem is solved in a second aspect by a product comprising
the mutant bispecific antibody of the first aspect;
optionally, a clearing composition; and
a targetable construct comprising a bivalent hapten, wherein both hapten moieties bind to the two scFvs on a single molecule of the mutant bispecific antibody, wherein the targetable construct further comprises a therapeutic cation, and/or one or more chelated or chemically bound therapeutic agents; as a combined preparation for simultaneous, separate or sequential use for treatment or diagnosis of a disease of a subject.

In an embodiment the product further comprises at least one therapeutic agent used to treat the disease or condition.

In an embodiment the targetable construct comprises an enzyme and which further comprises
a prodrug, wherein said enzyme is capable of converting said prodrug to a drug at the target site; or
a drug which is capable of being detoxified in said subject to form an intermediate of lower toxicity, wherein said enzyme is capable of reconverting said detoxified intermediate to a toxic form, and, increasing the toxicity of said drug at the target site; or
a prodrug which is activated in said subject through natural processes and is subject to detoxification by conversion to an intermediate of lower toxicity, wherein said enzyme is capable of reconverting said detoxified intermediate to a toxic form, and, increasing the toxicity of said drug at the target site.

In an embodiment the prodrug is selected from the group consisting of epirubicin glucuronide, CPT- 11, etoposide glucuronide, daunomicin glucuronide and doxorubicin glucuronide

In an embodiment the therapeutic agent is selected from the group consisting of ¹⁸F, ⁵²Fe, ⁶²Cu, ⁶⁴Cu, ⁶⁷Cu, ⁶⁷Ga, ⁶⁸Ga, ⁸⁶Y_{,} ⁸⁹Zr, ^{94m}Tc, ⁹⁴Tc, ^{99m}Tc, ¹¹¹In, ¹²³I, ¹²⁴I, ¹²⁵I, ¹³¹I, ¹⁵⁴⁻¹⁵⁸Gd, ¹⁷⁷Lu, ³²P, ¹⁸⁸Re, and ⁹⁰Y or a combination thereof.

In an embodiment the radioactive isotope is used to perform positron-emission tomography (PET).

In an embodiment the targetable construct comprises one or more image enhancing agents for use in magnetic resonance imaging (MRI).

In an embodiment the enhancing agent is selected from the group consisting of Mn, Fe and Gd.

In an embodiment the targetable construct comprises one or more image enhancing agents for use in ultrasound imaging.

In an embodiment the targetable construct is a liposome with a bivalent DTPA-peptide covalently attached to the outside surface of the liposome lipid membrane.

In an embodiment the liposome is gas filled.

In an embodiment the targetable construct comprises one or more radioactive isotopes useful for killing diseased tissue.

In an embodiment the energy range of the radioactive isotope is 60 to 700 keV.

In an embodiment the radioactive isotope is selected from the group consisting of ³²P, ³³P, ⁴⁷Sc, ⁶⁴Cu, ⁶⁷Cu, ⁶⁷Ga, ⁹⁰Y, ¹¹¹Ag, ¹¹¹In, ¹²⁵I, ¹³¹I ¹⁴²Pr, ¹⁵³Sm, ¹⁶¹Tb, ¹⁶⁶Dy, ¹⁶⁶Ho, ¹⁷⁷Lu, ¹⁸⁶Re, ¹⁸⁸Re, ¹⁸⁹Re , ²¹²Pb, ²¹²Bi, ²¹³Bi, ²¹¹At, ²²³Ra, ²²⁵Ac and a combination thereof.

In an embodiment the targetable construct comprises ¹⁰B atoms, and said treatment of a disease further comprises the step of irradiating said boron atoms localized at said diseased tissue, thereby effecting BNCT of said diseased tissue.

In an embodiment the therapeutic agent is a drug, toxin, hormone, enzyme, immunomodulator, chelator, boron compound, photoactive agent, dye, or radioisotope.

In an embodiment the targetable construct comprises one or more toxins.

In an embodiment the toxin is selected from the group consisting of ricin, abrin, ribonuclease, DNase 1, Staphylococcal enterotoxin-A, pokeweed antiviral protein, gelonin, diphtherin toxin, Pseudomonas exotoxin, Pseudomonas endotoxin and a combination thereof.

In an embodiment the targetable construct comprises one or more drugs.

In an embodiment the drug is selected from the group consisting of nitrogen mustards, ethylenimine derivatives, alkyl sulfonates, nitrosoureas, triazenes, folic acid analogs, anthracyclines, taxanes, COX- 2 inhibitors, pyrimidine analogs, purine analogs, antibiotics, enzymes, epipodophyllotoxins, platinum coordination complexes, vinca alkaloids, substituted ureas, methyl hydrazine derivatives, adrenocortical suppressants, antagonists, endostatin, taxols, camptothecins, doxorubicins and their analogs, and a combination thereof.

In an embodiment the targetable construct comprises one or more agents for photodynamic therapy.

In an embodiment the agent for photodynamic therapy is a photosensitizer.

In an embodiment the photosensitizer is selected from the group consisting of benzoporphyrin monoacid ring A (BPD-MA), tin etiopurpurin (SnET2), sulfonated aluminum phthalocyanine (AlSPc) and lutetium texaphyrin (Lutex).

In an embodiment the Fvs bind to a marker substance associated with a tumor, and wherein said tumor produces or is associated with antigens selected from the group consisting of colon-specific antigen-p (CSAp), carcinoembryonic antigen (CEA), CD4, CD5, CD8, CD14, CD15, CD19, CD20, CD21, CD22, CD23, CD25, CD33, CD37, CD38, CD40, CD40L, CD46, CD52, CD54, CD66a-e, CD74, CD75, CD80, CD126, B7, HLA-DR, Ia, Ii, HM1.24, MUC 1, MUC2, MUC3, MUC4,Tag-72, PSMA, EGP-1, EGP-2, PSA, AFP, HCG, HCG-beta, PLAP, PAP, histone, tenascin, VEGF, PIGF, S100, EGFR, insulin-like growth factor, HER2/neu, organotropic hormones, oncogene products, and cytokeratin.

In an embodiment the mutant bispecific antibody incorporates the Fv of a Class III anti-CEA antibody.

In an embodiment the mutant bispecific antibody incorporates the scFv of Mab 679.

In an embodiment the disease is an immune dysregulation disease, an autoimmune disease, organ graft rejection, cardiovascular disease, neurological disease or graft vs. host disease.

In an embodiment the autoimmune disease is selected from the group consisting of acute idiopathic thrombocytopenic purpura, chronic idiopathic thrombocytopenic purpura, dermatomyositis, Sydenham's chorea, myasthenia gravis, systemic lupus erythematosus, lupus nephritis, rheumatic fever, polyglandular syndromes, bullous pemphigoid, diabetes mellitus, Henoch-Schonlein purpura, poststreptococcalnephritis, erythema nodosurn, Takayasu's arteritis, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitisubiterans, Sjogren's syndrome, primary biliary cirrhosis, Hashimoto's thyroiditis, thyrotoxicosis, scleroderma, chronic active hepatitis, polymyositis/dermatomyositis, polychondritis, parnphigus vulgaris, Wegener's granulomatosis, membranous nephropathy, amyotrophic lateral sclerosis, tabes dorsalis, giant cell arteritis/polymyalgia, pernicious anemia, rapidly progressive glomerulonephritis and fibrosing alveolitis.

In an embodiment the disease is caused by a fungus, virus, parasite or bacterium, and the Fv of the mutant bispecific antibody targets the fungus, virus, parasite, or bacterium.

In an embodiment the virus is selected from the group consisting of human immunodeficiency virus (HIV), herpes virus, cytomegalovirus, rabies virus, influenza virus, hepatitis B virus, Sendai virus, feline leukemia virus, Reo virus, polio virus, human serum parvo-like virus, simian virus 40, respiratory syncytial virus, mouse mammary tumor virus, Varicella-Zoster virus, Dengue virus, rubella virus, measles virus, adenovirus, human T-cell leukemia viruses, Epstein-Barr virus, murine leukemia virus, mumps virus, vesicular stomatitis virus, Sindbis virus, lymphocytic choriomeningitis virus, wart virus and blue tongue virus.

In an embodiment the bacterium is selected from the group consisting of Anthrax bacillus, Streptococcus agalactiae, Legionella pneumopallidum, Lyme disease spirochetes, Pseudomonas aeruginosa, Mycobacterium leprae, Brucella abortus, Mycobacterium tuberculosis and Tetanus toxin.

In an embodiment the pathogen is a protozoan.

In an embodiment the protozoan is selected from the group consisting of Plasmodium falciparum, Plasmodium vivax, Toxoplasma gondii, Trypanosoma rangeli, Trypanosoma cruzi, Trypanosoma rhodesiensei, Trypanosoma brucei, Schistosoma mansoni, Schistosoma japanicum, Babesia bovis, Elmeria tenella, Onchocerca volvulus, Leishmania tropica, Trichinella spiralis, Onchocerca volvulus, Theileria parva, Taenia hydatigena, Taenia ovis, Taenia saginata, Echinococcus granulosus and Mesocestoides corti.

In an embodiment the parasite is a helminth or a malarial parasite.

In an embodiment the bacterium is mycoplasma.

In an embodiment the mycoplasina is selected from the group consisting of Mycoplasma arthritidis, M. hyorhinis, M. orale, M. arginini, Acholeplasma laidlawii, M. salivarum, and M. pneumoniae.

In an embodiment the fungus is selected from the group consisting of Histoplasma capsulatum, Blastomyces dermatitidis, Coccidioides immitis, and species of Candida.

In an embodiment the tissue is normal ectopic tissue.

In an embodiment the normal tissue is tissue from the ovary, thymus, parathyroid, bone marrow, or spleen.

In an embodiment the subject is mammalian.

In an embodiment the mammalian subject is a human or primate.

In an embodiment the mammalian subject is selected from the group consisting of rodents, lagamorphs, bovines, ovines, caprines, porcines, equines, canines, felines, domestic fowl, ungulates, and bear.

In an embodiment the product is for use in intraoperative diagnosis to identify diseased tissues.

In an embodiment the product is for use in endoscopic diagnosis to identify diseased tissues.

In an embodiment the product further comprises a second therapeutic agent for administration before, concurrently or after the prescribed diagnosis or treatment.

In an embodiment the second therapeutic agent is a drug, naked antibody, immunomodulator, or antibody conjugate bearing a drug, radioisotope, immunomodulator or toxin.

The problem is solved in a fourth aspect by a kit useful for treating or identifying diseased tissues in a subject comprising
the mutant bispecific antibody of the first aspect;
optionally, a clearing composition; and
a targetable construct comprising a bivalent hapten, wherein both hapten moieties bind to the two scFvs on a single molecule of the mutant bispecific antibody, wherein the targetable construct further comprises a therapeutic cation, and/or one or more chelated or chemically bound therapeutic agents; and optionally
a prodrug as defined herein

In an embodiment the immunomodulator is selected from the group consisting of a cytokine, a stem cell growth factor, a lymphotoxin, a hematopoietic factor, a colony stimulating factor (CSF), an interferon (IFN), erythropoietin, thrombopoietin and a combination thereof.

In an embodiment the lymphotoxin is tumor necrosis factor (TNF).

In an embodiment hematopoietic factor is interleukin (IL).

In an embodiment the colony stimulating factor is granulocytecolony stimulating factor (G-CSF) or granulocyte macrophage-colony stimulating factor (GM-CSF).

In an embodiment the interferon is interferon-α, -ß or -γ.

In an embodiment the stem cell growth factor is S1 factor.

In an embodiment the immunomodulator is IL-1, IL-2, IL-3, IL-6, IL-10, IL-12, IL-18, interferon-γ, TNF-α or a combination thereof.

In an embodiment the neurological disease is Alzheimer's Disease.

The problem is solved in a fifth aspect by the use of the mutant bispecific antibody of the first aspect, in the preparation of an agent for treatment or diagnosis.

### Brief Description of the Drawings

Figure 1 shows the heavy chain cDNA and amino acid sequences of hMN-14. The V_{H}, C_{H}1, Hinge, C_{H}2 and C_{H}3 regions are shown. The isoleucine at amino acid position 274 corresponds to isoleucine 253 according to the numbering system of Edelman, et al. See Edelman et al. Biochemistry 63, 78-85 (1969).
Figure 2 shows the light chain cDNA and amino acid sequences of hMN-14. The Vκ and Cκ regions are shown.
Figure 3 shows the biodistribution of hMN-14IgG^{1253A}-(734scFv)₂ in human colonic tumor-bearing mice, 1, 2, 3 and 4 days post injection. The designation "I253A" means that the isoleucine at position 253 is changed to an alanine. Data were expressed as a median percentage of injected dose per gram (% ID/g).
Figure 4 shows the biodistribution of hMN-14IgG-(734scFv)₂ in human colonic tumor-bearing mice, 1, 2, 3 and 4 days post injection. Data were expressed as a median percentage of injected dose per gram (% ID/g).

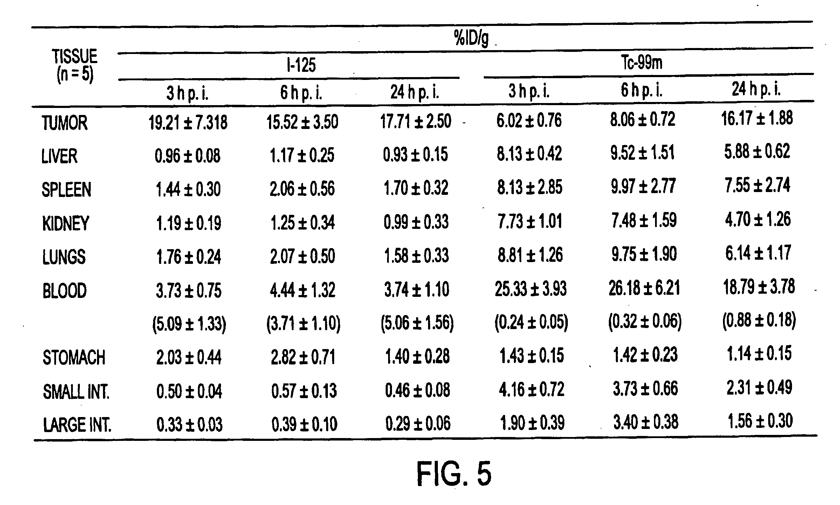

Figure 5 shows biodistribution data obtained from pretargeting experiments involving ¹²⁵I-hMN-14IgG-(734scFv)₂. The targetable construct was Tc-99m-labeled di-DTPA, IMP-192. Human colonic tumor-bearing mice were pretargeted with ¹²⁵I-hMN-14IgG-(734scFv)₂ for four days after which they were injected with a targetable conjugate. Data were obtained 3, 6 and 24 hours post injection of the targetable conjugate. Data are expressed as a median percentage of injected dose per gram (%ID/g). The tumor-to-blood ratio is reported under the entry for "Blood". The left side of the chart shows data for ¹²⁵I-labeled bsAb and the right side of the chart shows data for ^{99m}Tc-labeled targetable construct.

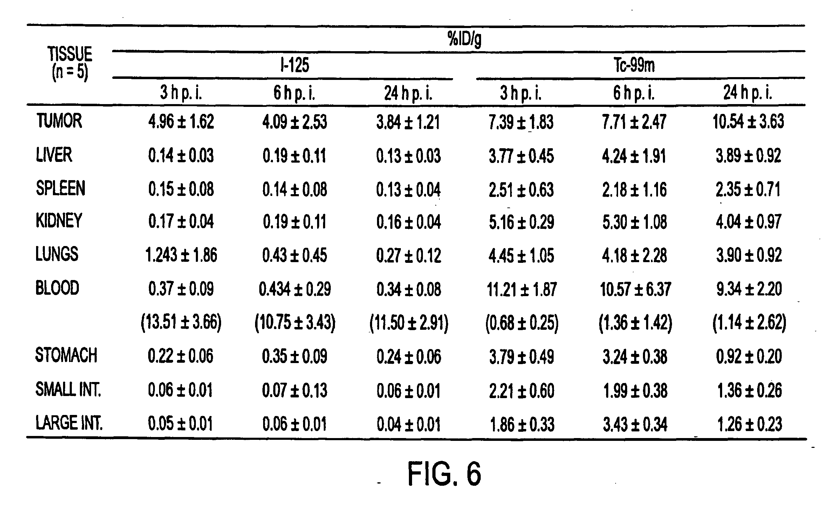

Figure 6 shows biodistribution data obtained from pretargeting experiments involving ¹²⁵I-hMN-14IgG-(734scFv)₂. The targetable construct was Tc-99m-labeled di-DTPA, IMP-192. Human colonic tumor-bearing mice were pretargeted with ¹²⁵I-hMN-14IgG-(734scFv)₂ for six days after which they were injected with a targetable conjugate. Data were obtained 3, 6 and 24 hours post injection of the targetable conjugate. Data are expressed as a median percentage of injected dose per gram (%ID/g). The tumor-to-blood ratio is reported under the entry for "Blood". The left side of the chart shows data for ¹²⁵I-labeled bsAb and the right side of the chart shows data for ^{99m}Tc-labeled targetable construct.

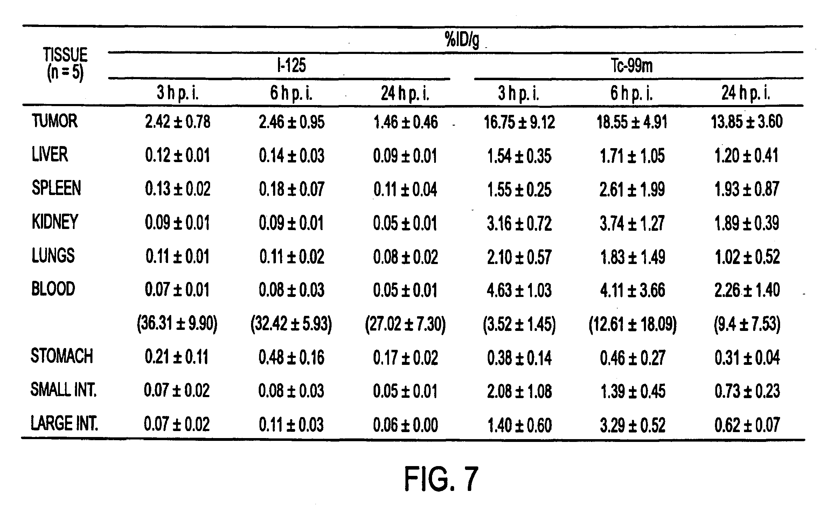

Figure 7 shows biodistribution data obtained from pretargeting experiments involving ¹²⁵I-hMN-14IgG^{1253A}-(734scFv)₂. The targetable construct was Tc-99m-labeled di-DTPA, IMP-192. Human colonic tumor-bearing mice were pretargeted with ¹²⁵I-hMN-14IgG^{1253A}-(734scFv)₂ for four days after which they were injected with a targetable conjugate. Data were obtained 3, 6 and 24 hours post injection of the targetable conjugate. Data are expressed as a median percentage of injected dose per gram (%ID/g). The tumor-to-blood ratio is reported under the entry for "Blood". The left side of the chart shows data for ¹²⁵I-labeled bsAb and the right side of the chart shows data for ^{99m}Tc-labeled targetable construct.
Figure 8 shows an ellution profile of a known standard of hMN-14IgG^{1253A}-(734scFv)₂ on a Bio-Sil SEC 250 300 mm x 7.8 mm HPLC column elluted at 1 mL/min with 0.2 M phosphate buffer pH 6.8.
Figure 9 shows an ellution profile of a known standard of Tc-99m IMP 192 on a Bio-Sil SEC 250 300 mm x 7.8 mm HPLC column elluted at I mL/min with 0.2 M phosphate buffer pH 6.8.
Figure 10 shows an ellution profile of a 1:1 mixture of hMN-14IgG^{1253A}-(734scFv)₂ to Tc-99m IMP 192 on a Bio-Sil SEC 250 300 mm x 7.8 mm HPLC column elluted at 1 mL/min with 0.2 M phosphate buffer pH 6.8.
Figure 11 shows an ellution profile of a 1:5 mixture of hMN-14IgG^{1253A}-(734scFv)₂ to Tc-99m IMP 192 on a Bio-Sil SEC 250 300 mm x 7.8 mm HPLC column elluted at 1 mL/min with 0.2 M phosphate buffer pH 6.8.
Figure 12 shows an ellution profile of a 20:1 mixture of hMN-14IgG^{1253A}-(734scFv)₂ to Tc-99m IMP 192 on a Bio-Sil SEC 250 300 mm x 7.8 mm HPLC column elluted at 1 mL/min with 0.2 M phosphate buffer pH 6.8.

### Detailed Description of the Preferred Embodiments

Unless otherwise specified, the terms "a" or "an" mean "one or more".

### I. Overview

The present invention relates to a mutant bsAb containing a human hinge constant region from IgG, two scFvs that each specifically binds to a binding site on a bivalent targetable construct such that a one to one binding interaction is obtained between the mutant bispecific antibody and the targetable construct and two Fvs, wherein said constant region contains one or more amino acid mutations in the CH2 domain, wherein said hinge constant region contains a mutation in which isoleucine at position 253 is replaced with alanine, or an amino acid other than leucine, and wherein the amino acid replacement enhances blood clearance equal to, or greater than replacement with alanine.

The mutant bsAb of the present invention clears a patient's body at a faster rate than the corresponding parent bsAb. Bispecifc antibodies are disclosed in WO 1999/66951. When employed in a pretargeting method, the amount of circulating primary targeting species (mutant bsAb not bound to the target site) is minimized. Additionally, the amount of targetable construct trapped in the blood is minimized.

The human hinge constant region may contain an effector function. The Fc portion of the antibody molecule provides effector functions, such as complement fixation and ADCC (antibody dependent cell cytotoxicity), which set mechanisms into action that may result in cell lysis. However, it is possible that the Fc portion is not required for therapeutic function, with other mechanisms, such as apoptosis, coming into play. Therefore, innate ADCC, apoptosis induction and complement activation/lysis may be achieved.

The scFvs are specific for a binding site on a targetable construct. The targetable construct is comprised of a carrier portion and at least 1 unit of a recognizable hapten. Examples of recognizable haptens include, but are not limited to, histamine succinyl glycine(HSG), DTPA and fluorescein isothiocyanate. The targetable construct may be conjugated to a variety of agents useful for treating or identifying diseased tissue. Examples of conjugated agents include, but are not limited to, chelators, metal chelate complexes, drugs, toxins (e.g., ricin, abrin, ribonuclease, DNase I, *Staphylococcal* enterotoxin-A, pokeweed antiviral protein, gelonin, diphtherin toxin, *Pseudomonas* exotoxin, *Pseudomonas* endotoxin) and other effector molecules. Suitable drugs for conjugation include doxorubicin analogs, SN-38, etoposide, methotrexate, 6-mercaptopurine or etoposide phosphate, calicheamicin, paclitaxel, 2-pyrrolinodoxorubicln, CC-1067, and adozelesin or a combination thereof. Exemplary drugs are nitrogen mustards, ethylenimine derivatives, alkyl sulfonates, nitrosoureas, triazenes, folic acid analogs, anthracyclines, taxanes, COX-2 inhibitors, pyrimidine analogs, purine analogs, antibiotics, enzymes, epipodophyllotoxins, platinum coordination complexes, vinca alkaloids, substituted ureas, methyl hydrazine derivatives, adrenocortical suppressants, antagonists, endostatin, taxols, camptothecins, doxorubicins and their analogs, and a combination thereof. Additionally, enzymes useful for activating a prodrug or increasing the target-specific toxicity of a drug can be conjugated to the targetable construct. Thus, the use of a mutant bsAb containing scFvs which are reactive to a targetable construct allows a variety of therapeutic and diagnostic applications to be performed without raising new bsAbs for each application.

Additionally, the present invention encompasses the bsAb of the present invention for use in a method for detecting or treating target cells, tissues or pathogens in a mammal, comprising administering an effective amount of a mutant bsAb comprising a human hinge constant region from IgG, two Fvs that each specifically binds to a binding site on a bivalent targetable construct such that a one to one binding interaction is obtained between the mutant bispecific antibody and the targetable construct and two scFvs, wherein said constant region contains one or more amino acid mutations in the C_{H}2 domain, wherein said hinge constant region contains a mutation in which isoleucine at position 253 is replaced with alanine, or an amino acid other than leucine, and wherein the amino acid replacement enhances blood clearance equal to, or greater than replacement with alanine.

As used herein, the term "pathogen" includes, but is not limited to fungi (e.g. Histoplasma capsulatum, Blastomyces dermatitidis, Coccidioides immitis, and species of Candida), viruses (e.g., human immunodeficiency virus (HIV), herpes virus, cytomegalovirus, rabies virus, influenza virus, hepatitis B virus, Sendai virus, feline leukemia virus, Reo virus, polio virus, human serum parvo-like virus, simian virus 40, respiratory syncytial virus, mouse mammary tumor virus, Varicella-Zoster virus, Dengue virus, rubella virus, measles virus, adenovirus, human T-cell leukemia viruses, Epstein-Barr virus, murine leukemia virus, mumps virus, vesicular stomatitis virus, Sindbis virus, lymphocytic choriomeningitis virus, wart virus and blue tongue virus), parasites, microbes (e.g. rickettsia) and bacteria (e.g., Streptococcus agalactiae, Legionella pneumophilia, Streptococcus pyogenes, Escherichia coli, Neisseria gonorrhoeae, Neisseria meningitidis, Pneumococcus, Hemophilis influenzae B, Treponema pallidum, Lyme disease spirochetes, Pseudomonas aeruginosa, Mycobacterium leprae, Brucella abortus, Mycobacterium tuberculosis, Anthrax spores and Tetanus toxin). *See* U.S. Patent No. 5,332,567.

As used herein, the term "antibody" refers to a full-length (i.e., naturally occurring or formed by normal immunoglobulin gene fragment recombinatorial processes) immunoglobulin molecule (e.g., an IgG antibody) or an immunologically active (i.e., specifically binding) portion of an immunoglobulin molecule, like an antibody fragment. The term antibody encompasses chimeric, cdr-grafted (humanized), and fully human antibodies. The term "IgG" is used to mean an antibody, i.e., an immunoglobulin G, generated against, and capable of binding specifically to an antigen. The term antibody is abbreviated as Ab. A monoclonal antibody is abbreviated as mAb.

A human antibody is an antibody obtained from transgenic mice that have been "engineered" to produce specific human antibodies in response to antigenic challenge. In this technique, elements of the human heavy and light chain locus are introduced into strains of mice derived from embryonic stem cell lines that contain targeted disruptions of the endogenous heavy chain and light chain loci. The transgenic mice can synthesize human antibodies specific for human antigens, and the mice can be used to produce human antibody-secreting hybridomas. Methods for obtaining human antibodies from transgenic mice are described by Green et al., Nature Genet. 7:13 (1994), Lonberg et al., Nature. 368:856 (1994), and Taylor et al., Int. Immun. 6:579 (1994). A fully human antibody also can be constructed by genetic or chromosomal transfection methods, as well as phage display technology, all of which are known in the art. See for example, McCafferty et al., Nature 348:552-553 (1990) for the production of human antibodies and fragments thereof in *vitro,* from immunoglobulin variable domain gene repertoires from unimmunized donors. In this technique, antibody variable domain genes are cloned in-frame into either a major or minor coat protein gene of a filamentous bacteriophage, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. In this way, the phage mimics some of the properties of the B cell. Phage display can be performed in a variety of formats, for their review, see e.g. Johnson and Chiswell, Current Opinion in Structural Biology 3:5564-571 (1993).

Human antibodies may also be generated by *in vitro* activated B cells. See U.S. Patent Nos. 5,567,610 and 5,229,27.

An antibody fragment is a portion of an antibody such as F(ab')₂, F(ab)₂, Fab', Fab, Fv, scFv and the like. Regardless of structure, an antibody fragment binds with the same antigen that is recognized by the intact antibody. For example, an anti-CEA monoclonal antibody fragment binds with an epitope of CEA.

The term "antibody fragment" also includes any synthetic or genetically engineered protein that acts like an antibody by binding to a specific antigen to form a complex. For example, antibody fragments include isolated fragments consisting of the light chain variable region, "Fv" fragments consisting of the variable regions of the heavy and light chains, recombinant single chain polypeptide molecules in which light and heavy variable regions are connected by a peptide linker ("scFv proteins"), and minimal recognition units consisting of the amino acid residues that mimic the hypervariable region.

A chimeric antibody is a recombinant protein that contains the variable domains and complementary determining regions derived from a first species, such as a rodent antibody, while the heavy and light chain constant regions of the antibody molecule is derived from a second species, such as a human antibody.

Humanized antibodies are recombinant proteins in which the complementarity determining regions of a monoclonal antibody have been transferred from heavy and light variable chains of a first species immunoglobulin, such as a murine immunoglobulin into the human heavy and lightvariable domains while the heavy and light chain constant regions of the antibody molecule is derived from a human antibody. Humanized antibodies are also referred to as CDR-grafted antibodies.

As used herein, the term "bispecific antibody" is an antibody capable of binding to two different moieties, i.e., a targeted tissue and a targetable construct.

As used herein, a therapeutic agent is a molecule or atom which is administered to a subject in combination according to a specific dosing schedule with the antibody of the present invention or conjugated to an antibody moiety to produce a conjugate which is useful for therapy. Examples of therapeutic agents include drugs, toxins, hormones, enzymes, immunomodulators, chelators, boron compounds, photoactive agents or dyes, and radioisotopes. Exemplary immunomodulators may be selected from the group consisting of a cytokine, a stem cell growth factor, a lymphotoxin, a hematopoietic factor, a colony stimulating factor (CSF), an interferon (IFN), erythropoietin, thrombopoietin and a combination thereof. Specifically useful are lymphotoxins , such as tumor necrosis factor (TNF), hematopoietic factors, such as interleukin (IL), colony stimulating factor, such as granulocyte-colony stimulating factor (G-CSF) or granulocyte macrophage-colony stimulating factor (GM-CSF)), interferon, such as interferons-α, -β or - γ, and stem cell growth factor, such as designated "S1 factor". More specifically, immunomodulator, such as IL-1, IL-2, IL-3, IL-6, IL-10, IL-12, IL-18, interferon-γ, TNF-α or a combination thereof are useful in the present invention.The term "scFv" is used to mean recombinant single chain polypeptide molecules in which light and heavy chain variable regions of an antibody are connected by a peptide linker.

The term "Fv" is used to mean fragments consisting of the variable regions of the heavy and light chains.

A "recombinant host" may be any prokaryotic or eukaryotic cell that contains either a cloning vector or expression vector. This term also includes those prokaryotic or eukaryotic cells, as well as an transgenic animal, that have been genetically engineered to contain the cloned gene(s) in the chromosome or genome of the host cell or cells of the host cells. Suitable mammalian host cells include myeloma cells, such as SP2/0 cells, and NS0 cells, as well as Chinese Hamster Ovary (CHO) cells, hybridoma cell lines and other mammalian host cell useful for expressing antibodies. Also particularly useful to express mAbs and other fusion proteins is a human cell line, PER.C6 disclosed in WO 0063403 A2, which produces 2 to 200-fold more recombinant protein as compared to conventional mammalian cell lines, such as CHO, COS, Vero, Hela, BHK and SP2- cell lines. Special transgenic animals with a modified immune system are particularly useful for making fully human antibodies.

The antigen may be any antigen. An exemplary antigen is a cell surface or tumor-associated antigen, or an antigen associated with a microorganism or parasite, or with a diseased tissue or cell type leading to disease, such as a B- or T-cell involved in autoimmune disease, or a target antigen of a cardiovascular or neurological disease (e.g., atherosclerotic plaque or embolus in the former and amyloid in the latter, such as associated with Alzheimer's disease). As used herein, the term "tissue" is used to mean a tissue as one of ordinary skill in the art would understand it to mean. As envisioned in the current application, tissue is also used to mean individual or groups of cells, or cell cultures, of a bodily tissue or fluid (e.g., blood cells). Furthermore, the tissue may be within a subject, or biopsied or removed from a subject. The tissue may also be a whole or any portion of a 1): using the bsAb bodily organ. Additionally, the tissue may be "fresh" in that the tissue would be recently removed from a subject without any preservation steps between the excision and the methods using the bsAb of the current invention. The tissue may also have been preserved by such standard tissue preparation techniques including, but not limited to, freezing, quick freezing, paraffin embedding and tissue fixation, prior to application of the methods of the current invention.

A "targeted tissue" is a system, organ, tissue, cell, receptor or organelle to which a targetable conjugate may be delivered. In the therapeutic aspects of the invention, the targeted tissue is infected, dysfunctional, displaced or ectopic (e.g., infected cells, cancer cells, endometriosis, etc.). Normal tissues, such as bone marrow, may also be ablated, as needed in a therapeutic intervention, by these methods. In diagnostic aspects of the invention, it is desired to detect the targeted tissue.

As used herein, the term "subject" refers to any animal (i.e., vertebrates and invertebrates) including, but not limited to humans and other primates, rodents (e.g., mice, rats, and guinea pigs), lagarmorphs (e.g., rabbits), bovines (e.g, cattle), ovines (e.g., sheep), caprines (e.g., goats), porcines (e.g., swine), equines (e.g., horses), canines (e.g., dogs), felines (e.g.. cats), domestic fowl (e.g., chickens, turkeys, ducks, geese, other gallinaceous birds, etc.), as well as feral or wild animals, including, but not limited to, such animals as ungulates (e.g., deer), bear, fish, lagarmorphs, rodents, birds, etc. h is not intended that the term be limited to a particular age or sex. Thus, adult and newborn subjects, as well as fetuses, whether male or female, are encompassed by the term.

As used herein, the term "parent bsAb" is used to mean a bsAb which is similar to a mutant bsAb in every way except that the hinge constant region of the parent bsAb does not contain one or more amino acid mutations in the C_{H}2-C_{H}3 domain interface region.

As used herein, the term "hinge constant region" comprises the C₁, C_{H}1, hinge, C_{H}2 and C_{H}3 regions of an IgG. The heavy chain constant region comprises the C_{H}1, hinge, C_{H}2 and C_{H}3 regions, while the light chain constant region comprises the C₁ region.

### II. The Mutant Bispecific Antibody

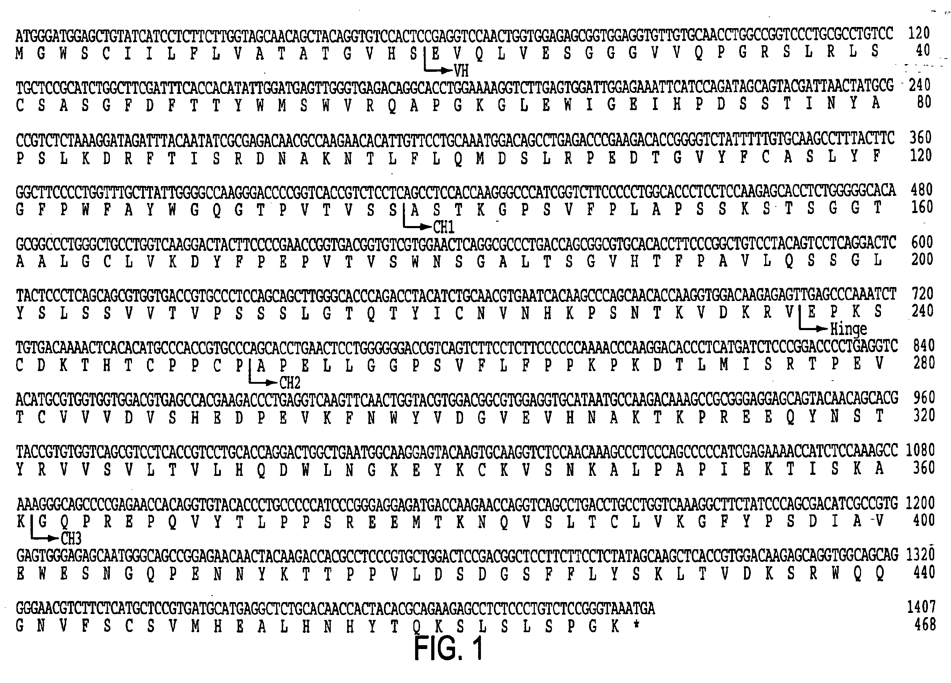

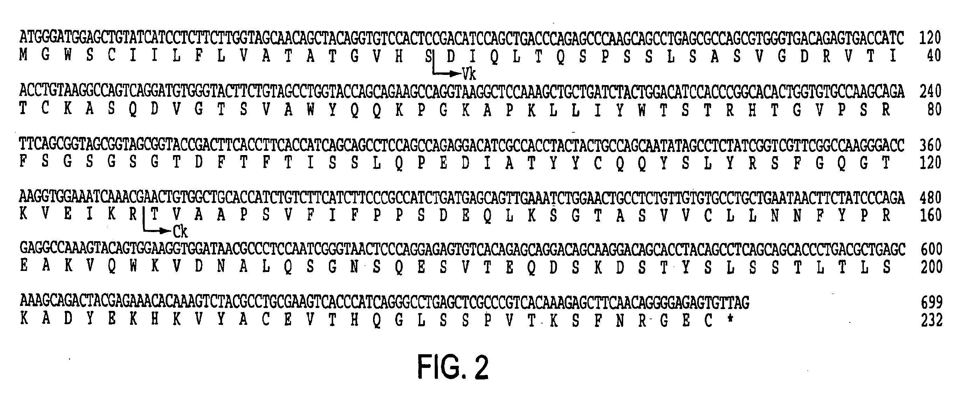

The Fvs of the mutant bsAb are derived from an antibody and specifically bind a targeted tissue. Exemplary Fvs are derived from anti-CD20 antibodies, such as those described in Provisional U.S. Application titled "Anti-CD20 Antibodies And Fusion Proteins Thereof And Methods Of Use", Attorney Docket No. 18733/1073, U.S. Provisional No. 60/356,132, U.S. Provisional Application No. 60/416,232 and Attorney Docket No. 18733/1155; hMN-14 antibodies, such as those disclosed in U.S. Application No. 5,874,540, which is a Class III anti-carcinoembryonic antigen antibody (anti-CEA antibody); Mu-9 antibodies, such as those described in WO 02/082041; LL1 antibodies, such as those described in U.S. Provisional Application No. 60/360,259; AFP antibodies, such as those described in U.S. Provisional Application No. 60/399,707; PAM4 antibodies, such as those described in Provisional U.S. Application titled "Monoclonal Antibody cPAM4", Attorney Docket No. 18733/1102; RS7 antibodies, such as those described in U.S. Provisional Application No. 60/360,229; and CD22 antibodies, such as those disclosed in U.S. Patent Nos. 5,789,554 and 6,187,287 and WO 1996/04925. Many other tumor-associated antigens of hematopoietic and solid tumors are known to those skilled in the art, as contained in the referenced applications, and include (but are not limited to) CD15, CD19, CD20, CD21, CD22, CD23, CD25, CD40, CD45, CD66, CD74, CD80, Ii, Ia, HLA-DR, PSMA, PSA, prostastic acid phosphatase, tenascin, Le(y), AFP, HCG, CEA, CSAp, PAM4, MUC1, MUC2, MUC3, MUC4, EGP-1, EGP-2, EGFR, HER2/*neu*, insulin growth-factor receptors, S100, VEGF, Placenta Growth Factor (PIGF), placental alkaline phosphatase, necrosis products, oncogene products, and the like. The heavy chain cDNA and amino acid sequences of hMN-14 are shown in Figure 1 and the light chain cDNA and amino acid sequences of hMN-14 are shown in Figure 2.

The cDNA encoding the Fvs may be inserted into a vector encoding the hinge constant region. An exemplary expression vector, pdHL2, which encodes the amino acids of the hinge constant region of IgG1 was reported by Gillies S.D., Lo KM, and Wesolowski, J. J. Immunol Methods 125 191-202 (1989) and Losman, M.J. et al. Cancer Supplement 80 2660-2666 (1997) and may be used to construct mutant bispecific antibodies of the present invention.

The Fvs can be from murine antibodies, cdr-grafted (humanized) antibodies, or human antibodies. The Fvs can be derived from human monoclonal antibodies, transgenic mice with human Fv-libraries, or phage/ribosome human IgG libraries.

When the Fvs are derived from CDR-grafted antibodies, appropriate variable region framework sequences may be used having regard to the class or type of the donor antibody from which the antigen binding regions are derived. Preferably, the type of human framework used is of the same or similar class or type as the donor antibody. Advantageously, the framework is chosen to maximize or optimize homology with the donor antibody sequence, particularly at positions spatially close to or adjacent the CDRs. Examples of human frameworks which may be used to construct CDR-grafted antibodies are LAY, POM, TUR, TEI, KOL, NEWM, REI and EU (Kabat et al, 1987). KOL and NEWM and are suitable for heavy chain construction. REI is suitable for light chain construction and EU is suitable for both heavy chain and light chain construction.

The light or heavy chain variable regions of the CDR-grafted antibodies may be fused to human light or heavy chain constant domains as appropriate (the term "heavy chain constant domains" as used herein is to be understood to include hinge regions unless specified otherwise). The human constant domains of the CDR-grafted antibodies, where present, may be selected having regard to the proposed function of the antibody, in particular, the effector functions which may be required. For example, IgG1 and IgG3 isotype domains may be used when the CDR-grafted antibody is intended for therapeutic purposes and antibody effector functions are required. Alternatively, IgG2 and IgG4 isotype domains may be used when the CDR-grafted antibody is intended for purposes for which antibody effector functions are not required, e.g., for imaging, diagnostic or cytotoxic targeting purposes. Light chain human constant domains which may be fused to the light chain variable region include human Lambda or, especially, human Kappa chains.

It is disclosed that the hinge constant region of the bi-specific mutant antibody contains one or more amino acid mutations in the C_{H}2-C_{H}3 domain interface region. In other words, when the human hinge constant region of the bi-specific mutant antibody is compared to the human hinge constant region of the bi-specific parent antibody, the regions will differ by one or more amino acids.

A mutation may encompass, for example, a "conservative" change, wherein a substituted amino has similar structural or chemical properties, such as charge or size (e.g., replacement of leucine with isoleucine). A mutation also encompasses, for example, a "non-conservative" change (e.g., replacement of a glycine with a tryptophan).

In the bsAb of the invention, the amino acid at position 253 (according to the numbering system of Edelman) is mutated. An exemplary mutation at this position replacing isoleucine with alanine. In some embodiments, the amino acid at position 253 is mutated to an amino acid wherein the pharmacokinetics of clearance of the mutant bsAb are similar to that observed when the amino acid at position 253 is changed to alanine.

In one embodiment, the hinge constant region of the bi-specific mutant antibody comprises the amino acid sequences of human IgG1. The amino acids encoding the C_{H}1, hinge, C_{H}2 and C_{H}3 regions of the heavy chain are shown as amino acid numbers 139-468 of Figure 1, while the amino acids encoding the C₁ chain are shown as amino acid numbers 128-232 of Figure 2. It is noted that the numbering system used to identify isoleucine 253 is consistent with the numbering system used by Edelman et al. in their disclosure of the Eu heavy and light chains. Edelman et al. Biochemistry 63, 78-85 (1969).

The scFv component of the bi-specific mutant antibody specifically binds to a binding site on a bivalent targetable construct. The use of any scFv component is contemplated by the present invention. Preferred scFv components are 679 scFv (derived from a murine anti-HSG) and 734scFv (derived from a murine anti-diDTPA). The scFv can be murine, cdr-grafted (humanized) or human.

When the scFvs are derived from CDR-grafted antibodies, appropriate variable region framework sequences may be used having regard to the class or type of the donor antibody from which the antigen binding regions are derived. Preferably, the type of human framework used is of the same or similar class or type as the donor antibody. Advantageously, the framework is chosen to maximize or optimize homology with the donor antibody sequence, particularly at positions spatially close to or adjacent the CDRs. Examples of human frameworks which may be used to construct CDR-grafted antibodies are LAY, POM, TUR, TEI, KOL, NEWM, REI and EU (Kabat et al, 1987). KOL and NEWM and are suitable for heavy chain construction. REI is suitable for light chain construction and EU is suitable for both heavy chain and light chain construction.

The light or heavy chain variable regions of the CDR-grafted antibodies may be fused to human light or heavy chain constant domains as appropriate, (the term "heavy chain constant domains" as used herein are to be understood to include hinge regions unless specified otherwise). The human constant domains of the CDR-grafted antibodies, where present, may be selected having regard to the proposed function of the antibody, in particular the effector functions which may be required. For example, IgG1 and IgG3 isotype domains may be used when the CDR-grafted antibody is intended for therapeutic purposes and antibody effector functions are required. Alternatively, IgG2 and IgG4 isotype domains may be used when the CDR-grafted antibody is intended for purposes for which antibody effector functions are not required, e.g. for imaging, diagnostic or cytotoxic targeting purposes. Light chain human constant domains which may be fused to the light chain variable region include human Lambda or, especially, human Kappa chains.

A preferred mutant bsAb is hMN-14IgG^{I253A}-(734scFv)₂. In this mutant bsAb, the FVs are derived from hMN-14IgG, the scFvs are 734scFV (derived from a murine anti-diDTPA) and the hinge constant region comprises the amino acid sequences of human IgG1.

According to the invention, a one to one binding interaction is obtained between the mutant bsAb and a targetable construct. For example, when the mutant bsAb of the present invention interacts with the bivalent targetable construct IMP 192 which contains two DTPA sites, one bsAb binds to one IMP 192. This interaction is illustrated by Example 3.

### III. Constructs Targetable to the Mutant bsAb

The mutant bsAb of the present invention binds a targetable construct whereby the scFvs of the mutant bsAb each specifically binds to a binding site on a bivalent targetable construct such that a one to one binding interaction is obtained between the mutant bispecific antibody and the targetable construct. The targetable construct can be of diverse structure, but is selected not only to elicit sufficient immune responses, but also for rapid in *vivo* clearance. Exemplary targetable constructs for use in the present application are described in WO 1999/66951 and WO 02/082041.

Hydrophobic agents are best at eliciting strong immune responses, whereas hydrophilic agents are preferred for rapid *in vivo* clearance, thus, a balance between hydrophobic and hydrophilic needs to be established. This is accomplished, in part, by relying on the use of hydrophilic chelating agents to offset the inherent hydrophobicity of many organic moieties. Also, sub-units of the targetable construct may be chosen which have opposite solution properties, for example, peptides, which contain amino acids, some of which are hydrophobic and some of which are hydrophilic. Aside from peptides, carbohydrates may be used.

Peptides having as few as two amino-acid residues may be used, preferably two to ten residues, if also coupled to other moieties, such as chelating agents. The linker should be a low molecular weight conjugate, preferably having a molecular weight of less than 50,000 daltons, and advantageously less than about 20,000 daltons, 10,000 daltons or 5,000 daltons, including the metal ions in the chelates. For instance, the known peptide DTPA-Tyr-Lys(DTPA)-OH (wherein DTPA is diethylenethaminepentaacetic acid) has been used to generate antibodies against the indium-DTPA portion of the molecule. However, by use of the non-indium-containing molecule, and appropriate screening steps, new Abs against the tyrosyl-lysine dipeptide can be made. More usually, the antigenic peptide will have four or more residues, such as the peptide DOTA-Phe-Lys(HSG)-Tyr-Lys(HSG)-NH₂, wherein DOTA is 1,4,7,10-tetraazacyclododecanetetraacetic acid and HSG is the histamine succinyl glycyl group of the formula:

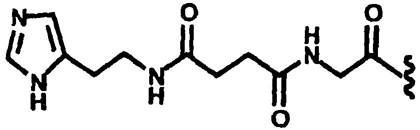

The non-metal-containing peptide may be used as an immunogen, with resultant Abs screened for reactivity against the Phe-Lys-Tyr-Lys backbone.

The invention also contemplates the incorporation of unnatural amino acids, e.g., D-amino acids, into the backbone structure to ensure that, when used with the final bsAb/linker system, the scFv component which recognizes the linker moiety is completely specific. The invention further contemplates other backbone structures such as those constructed from non-natural amino acids and peptoids.

The peptides to be used as immunogens are synthesized conveniently on an automated peptide synthesizer using a solid-phase support and standard techniques of repetitive orthogonal deprotection and coupling. Free amino groups in the peptide, which are to be used later for chelate conjugation, are advantageously blocked with standard protecting groups such as an acetyl group. Such protecting groups will be known to the skilled artisan. *See* Greene and Wuts Protective Groups in Organic Synthesis, 1999 (John Wiley and Sons, N.Y.). When the peptides are prepared for later use the mutant bsAb, they are advantageously cleaved from the resins to generate the corresponding C-terminal amides, in order to inhibit *in vivo* carboxypeptidase activity.

The haptens of the immunogen comprise an immunogenic recognition moiety, for example, a chemical hapten. Using a chemical hapten, preferably the HSG or DTPA hapten, high specificity of the linker for the antibody is exhibited. This occurs because antibodies raised to the HSG or DTPA hapten are known and the scFv portion of the antibody can be easily incorporated into the mutant bsAb. Thus, binding of the linker with the attached hapten would be highly specific for the scFv component.

The targetable construct may be monovalent or bivalent, with bivalent peptides being the preferred peptide. One exemplary targetable construct is IMP 192 (Ac-Lys(DTPA)-Tyr-Lys(DTPA)-Lys(TscG-Cys-)-NH₂). IMP 192 binds both Tc-99m and In-111 for diagnosis, and Re-188 and Re-186 for therapy. IMP 192 also binds bivalent DTPA-peptides with tyrosine.

The targetable construct as bound by the bsAb of the invention may comprise one or more radioactive isotopes useful for detecting diseased tissue. Particularly useful diagnostic radionuclides include, but are not limited to, ¹⁸F, ⁵²Fe,⁶²Cu, ⁶⁴Cu, ⁶⁷Cu, ⁶⁷Ga, ⁶⁸Ga, ⁸⁶Y, ⁸⁹Zr, ^{94m}Tc, ⁹⁴Tc, ^{99m}Tc, ¹¹¹In, ¹²³I, ¹²⁴I, ¹²⁵I, ¹³¹I, ¹⁵⁴⁻¹⁵⁸Gd, ¹¹⁷Lu, ³²P, ¹⁸⁸Re, ⁹⁰Y, or other gamma-, beta-, or positron-emitters, preferably with an energy in the range of 20 to 4,000 keV, more preferably in the range of 25 to 4,000 keV, and even more preferably in the range of 20 to 1,000 keV, and still more preferably in the range of 70 to 700 keV.

The targetable construct as bound by the bsAb of the invention may comprise one or more radioactive isotopes useful for treating diseased tissue. Particularly useful therapeutic radionuclides include, but are not limited to ³²P, ¹³P, ⁴⁷Sc, ⁶⁴Cu, ⁶⁷Cu, ⁶⁷Ga, ⁹⁰Y, ¹¹¹Ag, ¹¹¹In, ¹²⁵I, ¹³¹I, ¹⁴²Pr, ¹⁵³Sm, ¹⁶¹Tb, ¹⁶⁶Dy, ¹⁶⁶Ho, ¹⁷⁷Lu, ¹⁸⁶Re, ¹⁸⁸Re, ¹⁸⁹Re, ²¹²Pb, ²¹²Bi, ²¹³Bi, ²¹¹At, ²²³Ra and ²²⁵Ac. The therapeutic radionuclide preferably has an energy in the range of 60 to 700 keV.

The targetable construct as bound by the bsAb of the invention may comprise one or more image enhancing agents for use in magnetic resonance imaging (MRI). By way of non-limiting example, the targetable compound comprises one or more paragmagnetic ions, such as Mn, Fe, and Gd.

The targetable construct as bound by the bsAb of the invention may comprise one or more image enhancing agents for use in ultrasound. By way of non-limiting example, the targetable construct comprises one or more ultrasound imaging agents. In one such embodiment, the targetable construct is a liposome with a bivalent DTPA-peptide covalently attached to the outside surface of the liposome lipid membrane. Optionally, said liposome may be gas filled.

### IV. Chelate Moieties

The presence of hydrophilic chelate moieties on the linker moieties helps to ensure rapid *in vivo* clearance. In addition to hydrophilicity, chelators are chosen for their metal-binding properties, and are changed at will since, at least for those linkers whose bsAb epitope is part of the peptide or is a non-chelate chemical hapten, recognition of the metal-chelate complex is no longer an issue.

Particularly useful metal-chelate combinations include 2-benzyl-DTPA and its monomethyl and cyclohexyl analogs, used with ⁴⁷Sc, ⁵²Fe, ⁵⁵Co, ⁶⁷Ga, ⁶⁸Ga, ¹¹¹In, ⁸⁹Zr, ⁹⁰Y, ¹⁶¹Tb, ¹⁷⁷Lu, ²¹²Bi, ²¹³Bi, and ²²⁵Ac for radio-imaging and RAIT. The same chelators, when complexed with non-radioactive metals, such as Mn, Fe and Gd can be used for MRI, when used along with the mutant bsAbs of the invention. Macrocyclic chelators such as NOTA (1,4,7-triaza-cyclononane-N,N',N"-triacetic acid), DOTA, and TETA (p-bromoacetamido-benzyl-tetraethylaminetetraacetic acid) are of use with a variety of metals and radiometals, most particularly with radionuclides of Ga, Y and Cu, respectively.

DTPA and DOTA-type chelators, where the ligand includes hard base chelating functions such as carboxylate or amine groups, are most effective for chelating hard acid cations, especially Group IIa and Group IIIa metal cations. Such metal-chelate complexes can be made very stable by tailoring the ring size to the metal of interest. Other ring-type chelators such as macrocyclic polyethers are of interest for stably binding nuclides such as ²²³Ra for RAIT. Porphyrin chelators may be used with numerous radiometals, and are also useful as certain cold metal complexes for bsAb-directed immuno-phototherapy. More than one type of chelator may be conjugated to a carrier to bind multiple metal ions, *e.g.,* cold ions, diagnostic radionuclides and/or therapeutic radionuclides. Particularly useful therapeutic radionuclides include, but are not limited to ³²P, ¹³P, ⁴⁷Sc, ⁶⁴Cu, ⁶⁷Cu, ⁶⁷Ga, ⁹⁰Y, ¹¹¹Ag, ¹¹¹In, ¹²⁵I, ¹³¹I, ¹⁴²Pr, ¹⁵³Sm, ¹⁶¹Tb, ¹⁶⁶Dy, ¹⁶⁶Ho, ¹⁷⁷Lu, ¹⁸⁶Re, ¹⁸⁸Re, ¹⁸⁹Re, ²¹²Pb, ²¹²Bi, ²¹³Bi, ²¹¹At, ²²³Ra and ²²⁵Ac. Particularly useful diagnostic radionuclides include, but are not limited to, ¹⁸F, ⁵²Fe, ⁶²Cu, ⁶⁴Cu, ⁶⁷Cu, ⁶⁷Ga, ⁶⁸Ga, ⁸⁶Y, ⁸⁹Zr, ^{94m}Tc, ⁹⁴Tc, ^{99m}Tc, ¹¹¹In, ¹²³I, ¹²⁴I, ¹²⁵I, ¹³¹I, ¹⁵⁴⁻¹⁵⁸Gd and ¹⁷⁵Lu.

Chelators such as those disclosed in U.S. Patent 5,753,206, especially thiosemi-carbazonylglyoxylcysteine(Tscg-Cys) and thiosemicarbazinyl-acetylcysteine (Tsca-Cys) chelators are advantageously used to bind soft acid cations of Tc, Re, Bi and other transition metals, lanthanides and actinides that are tightly bound to soft base ligands, especially sulfur- or phosphorus-containing ligands. It can be useful to link more than one type of chelator to a peptide, e.g., a DTPA or similar chelator for, say In(III) cations, and a thiol-containing chelator, e.g., Tscg-Cys, for Tc cations. Because antibodies to a di-DTPA hapten are known (Barbet '395, *supra*) and are readily coupled to a targeting antibody to form a bsAb, it is possible to use a peptide hapten with cold diDTPA chelator and another chelator for binding a radioisotope, in a pretargeting protocol, for targeting the radioisotope. One example of such a peptide is Ac-Lys(DTPA)-Tyr-Lys(DTPA)-Lys(Tscg-Cys-)-NH₂. This peptide can be preloaded with In(III) and then labeled with 99m-Tc cations, the In(III) ions being preferentially chelated by the DTPA and the Tc cations binding preferentially to the thiol-containing Tscg-Cys. Other hard acid chelators such as NOTA, DOTA, TETA and the like can be substituted for the DTPA groups, and Mabs specific to them can be produced using analogous techniques to those used to generate the anti-di-DTPA Mab.

It will be appreciated that two different hard acid or soft acid chelators can be incorporated into the linker, e.g., with different chelate ring sizes, to bind preferentially to two different hard acid or soft acid cations, due to the differing sizes of the cations, the geometries of the chelate rings and the preferred complex ion structures of the cations. This will permit two different metals, one or both of which may be radioactive or useful for MRI enhancement, to be incorporated into a linker for eventual capture by a pretargeted bsAb.

Preferred chelators include NOTA, DOTA and Tscg and combinations thereof. These chelators have been incorporated into a chelator-peptide conjugate motif as exemplified in the following constructs:
(a) DOTA-Phe-Lys(HSG)-D-Tyr-Lys(HSG)-NH₂;
(b) DOTA-Phe-Lys(HSG)-Tyr-Lys(HSG)-NH₂;
(c) Ac-Lys(HSG)D-Tyr-Lys(HSG)-Lys(Tscg-Cys)-NH₂;
(d) 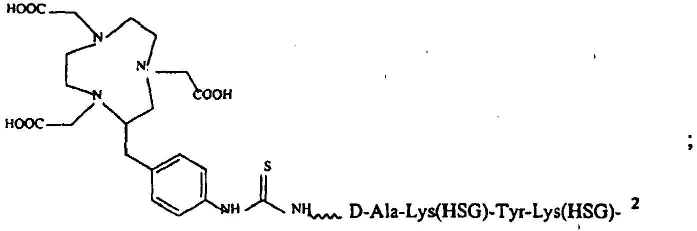 and
(e)

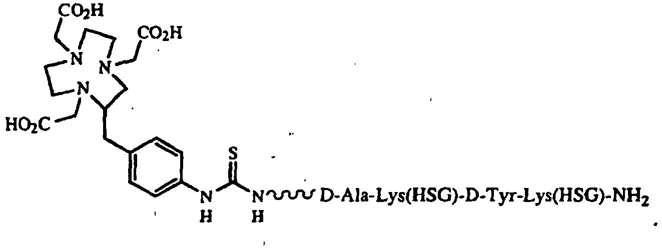

The chelator-peptide conjugates (d) and (e), above, has been shown to bind ⁶⁸Ga and is thus useful in positron emission tomography (PET) applications.

Chelators are coupled to the linker moieties using standard chemistries which are discussed more fully in the working Examples below. Briefly, the synthesis of the peptide Ac-Lys(HSG)D-Tyr-Lys(HSG)-Lys(Tscg-Cys-)-NH₂ was accomplished by first attaching Aloc-Lys(Fmoc)-OH to a Rink amide resin on the peptide synthesizer. The protecting group abbreviations "Aloc" and "Fmoc" used herein refer to the groups allyloxycarbonyl and fluorenylmethyloxy carbonyl. The Fmoc-Cys(Trt)-OH and TscG were then added to the side chain of the lysine using standard Fmoc automated synthesis protocols to form the following peptide: Aloc-Lys(Tscg-Cys(Trt)-rink resin. The Aloe group was then removed. The peptide synthesis was then continued on the synthesizer to make the following peptide: (Lys(Aloc)-D-Tyr-Lys(Aloc)-Lys(Tscg-Cys(Trt)-)-rink resin. Following N-terminus acylation, and removal of the side chain Aloc protecting groups. The resulting peptide was then treated with activated N-trityl-HSG-OH until the resin gave a negative test for amines using the Kaiser test. *See* Karacay et al. Bioconjugate Chem. 11:842-854 (2000). The synthesis of Ac-Lys(HSG)D-Tyr-Lys(HSG)-Lys(Tscg-Cys-)-NH₂, as well as the syntheses of DOTA-Phe-Lys(HSG)-D-Tyr-Lys(HSG)-NH₂; and DOTA-Phe-Lys(HSG)-Tyr-Lys(HSG)-NH₂ are described in greater detail below.

### V. General Methods for Preparation of Metal Chelates

Chelator-peptide conjugates may be stored for long periods as solids. They may be metered into unit doses for metal-binding reactions, and stored as unit doses either as solids, aqueous or semi-aqueous solutions, frozen solutions or lyophilized preparations. They may be labeled by well-known procedures. Typically, a hard acid cation is introduced as a solution of a convenient salt, and is taken up by the hard acid chelator and possibly by the soft acid chelator. However, later addition of soft acid cations leads to binding thereof by the soft acid chelator, displacing any hard acid cations which may be chelated therein. For example, even in the presence of an excess of cold ¹¹¹InCl₃, labeling with 99m-Tc(V) glucoheptonate or with Tc cations generated *in situ* with stannous chloride and Na99m-TcO₄ proceeds quantitatively on the soft acid chelator. Other soft acid cations such as ¹⁸⁶Re, ¹⁸⁸Re, ²¹³Bi and divalent or trivalent cations of Mn, Co, Ni, Pb, Cu, Cd, Au, Fe, Ag (monovalent), Zn and Hg, especially ⁶⁴Cu and ⁶⁷Cu, and the like, some of which are useful for radioimmunodiagnosis or radioimmunotherapy, can be loaded onto the linker peptide by analogous methods. Re cations also can be generated *in situ* from perrhenate and stannous ions or a prereduced rhenium glucoheptonate or other transchelator can be used. Because reduction of perrhenate requires more stannous ion (typically above 200 µg/mL final concentration) than is needed for the reduction of Tc, extra care needs to be taken to ensure that the higher levels of stannous ion do not reduce sensitive disulfide bonds such as those present in disulfide-cyclized peptides. During radiolabeling with rhenium, similar procedures are used as are used with the Tc-99m. A preferred method for the preparation of ReO metal complexes of the Tscg-Cys- ligands is by reacting the peptide with ReOCl₃(P(Ph₃)₂ but it is also possible to use other reduced species such as ReO(ethylenediamine)₂.

### VI. Methods for Raising Antibodies

Antibodies to peptide backbones are generated by well-known methods for Ab production. For example, injection of an immunogen, such as (peptide)ₙ-KLH, wherein KLH is keyhole limpet hemocyanin, and n=1-30, in complete Freund's adjuvant, followed by two subsequent injections of the same immunogen suspended in incomplete Freund's adjuvant into immunocompetent animals, is followed three days after an i.v. boost of antigen, by spleen cell harvesting. Harvested spleen cells are then fused with Sp2/0-Ag14 myeloma cells and culture supernatants of the resulting clones analyzed for anti-peptide reactivity using a direct-binding ELISA. Fine specificity of generated Abs can be analyzed for by using peptide fragments of the original immunogen. These fragments can be prepared readily using an automated peptide synthesizer. For Ab production, enzyme-deficient hybridomas are isolated to enable selection of fused cell lines. This technique also can be used to raise antibodies to one or more of the chelates comprising the linker, e.g., In(III)-DTPA chelates. Monoclonal mouse antibodies to an In(III)-di-DTPA are known (Barbet '395 *supra*).

The mutant bispecific antibodies used in the present invention are specific to a variety of cell surface or intracellular tumor-associated antigens as marker substances. These markers may be substances produced by the tumor or may be substances which accumulate at a tumor site, on tumor cell surfaces or within tumor cells, whether in the cytoplasm, the nucleus or in various organelles or sub-cellular structures. Among such tumor-associated markers are those disclosed by Herberman, "Immunodiagnosis of Cancer", in Fleisher ed., "The Clinical Biochemistry of Cancer", page 347 (American Association of Clinical Chemists, 1979) and in U.S. Patent Nos. 4,150,149; 4,361,544; and 4,444,744.

Tumor-associated markers have been categorized by Herberman, *supra,* in a number of categories including oncofetal antigens, placental antigens, oncogenic or tumor virus associated antigens, tissue associated antigens, organ associated antigens, ectopic hormones and normal antigens or variants thereof. Occasionally, a sub-unit of a tumor-associated marker is advantageously used to raise antibodies having higher tumor-specificity, e.g., the beta-subunit of human chorionic gonadotropin (HCG) or the gamma region of carcino embryonic antigen (CEA), which stimulate the production of antibodies having a greatly reduced cross-reactivity to non-tumor substances as disclosed in U.S. Patent Nos. 4,361,644 and 4,444,744.

Another marker of interest is transmembrane activator and CAML-interactor (TACI). *See* Yu et al. Nat. Immunol. I:252-256 (2000). Briefly, TACI is a marker for B-cell malignancies (e.g., lymphoma). Further it is known that TACI and B cell maturation antigen (BCMA) are bound by the tumor necrosis factor homolog a proliferation-inducing ligand (APRIL). APRIL stimulates in *vitro* proliferation of primary B and T cells and increases spleen weight due to accumulation of B cells *in vivo.* APRIL also competes with TALL-1 (also called BLyS or BAFF) for receptor binding. Soluble BCMA and TACI specifically prevent binding of APRIL and block APRIL-stimulated proliferation of primary B cells. BCMA-Fc also inhibits production of antibodies against keyhole limpet hemocyanin and Pneumovax in mice, indicating that APRIL and/or TALL-1 signaling via BCMA and/or TACI are required for generation of humoral immunity. Thus, APRIL-TALL-1 and BCMA-TACI form a two ligand-two receptor pathway involved in stimulation of B and T cell function.

After the initial raising of antibodies to the immunogen, the antibodies can be sequenced and subsequently prepared by recombinant techniques. Humanization and chimerization of murine antibodies and antibody fragments are well known to those skilled in the art. For example, humanized monoclonal antibodies are produced by transferring mouse complementary determining regions from heavy and light variable chains of the mouse immunoglobulin into a human variable domain, and then, substituting human residues in the framework regions of the murine counterparts. The use of antibody components derived from humanized monoclonal antibodies obviates potential problem associated with the immunogenicity of murine constant regions. General techniques for cloning murine immunoglobulin variable domains are described, for example, by the publication of Orlandi et al., Proc. Nat'l Acad. Sci. USA 86: 3833 (1989) Techniques for producing humanized Mabs are described, for example, by Jones et al., Nature 321: 522 (1986), Riechmann et al., Nature 332: 323 (1988), Verhoeyen et al., Science 239: 1534 (1988), Carter et al., Proc. Nat'l Acad. Sci. USA 89: 4285 (1992), Sandhu, Crit. Rev. Biotech. 12: 437 (1992), and Singer et al., J. Immun. 150: 2844 (1993)

Alternatively, fully human antibodies can be obtained from transgenic non-human animals. See, e.g., Mendez et al., Nature Genetics, 15: 146-156 (1997); U.S. Patent No. 5,633,425. For example, human antibodies can be recovered from transgenic mice possessing human immunoglobulin loci. The mouse humoral immune system is humanized by inactivating the endogenous immunoglobulin genes and introducing human immunoglobulin loci. The human immunoglobulin loci are exceedingly complex and comprise a large number of discrete segments which together occupy almost 0.2% of the human genome. To ensure that transgenic mice are capable of producing adequate repertoires of antibodies, large portions of human heavy- and light-chain loci must be introduced into the mouse genome. This is accomplished in a stepwise process beginning with the formation of yeast artificial chromosomes (YACs) containing either human heavy-or light-chain immunoglobulin loci in germline configuration. Since each insert is approximately 1 Mb in size, YAC construction requires homologous recombination of overlapping fragments of the immunoglobulin loci. The two YACs, one containing the heavy-chain loci and one containing the light-chain loci, are introduced separately into mice via fusion of YAC-containing yeast spheroblasts with mouse embryonic stem cells. Embryonic stem cell clones are then microinjected into mouse blastocysts. Resulting chimeric males are screened for their ability to transmit the YAC through their germline and are bred with mice deficient in murine antibody production. Breeding the two transgenic strains, one containing the human heavy-chain loci and the other containing the human light-chain loci, creates progeny which produce human antibodies in response to immunization.

Unrearranged human immunoglobulin genes also can be introduced into mouse embryonic stem cells via microcell-mediated chromosome transfer (MMCT). See, e.g., Tomizuka et al., Nature Genetics, 16: 133 (1997). In this methodology microcells containing human chromosomes are fused with mouse embryonic stem cells. Transferred chromosomes are stably retained, and adult chimeras exhibit proper tissue-specific expression.

As an alternative, an antibody or antibody fragment of the present invention may be derived from human antibody fragments isolated from a combinatorial immunoglobulin library. See, e.g., Barbas et al., METHODS: A Companion to Methods in Enzymology 2: 119 (1991), and Winter et al., Ann. Rev. Immunol. 12: 433 (1994) Many of the difficulties associated with generating monoclonal antibodies by B-cell immortalization can be overcome by engineering and expressing antibody fragments in *E. coli,* using phage display. To ensure the recovery of high affinity, monoclonal antibodies a combinatorial immunoglobulin library must contain a large repertoire size. A typical strategy utilizes mRNA obtained from lymphocytes or spleen cells of immunized mice to synthesize cDNA using reverse transcriptase. The heavy- and light-chain genes are amplified separately by PCR and ligated into phage cloning vectors. Two different libraries are produced, one containing the heavy-chain genes and one containing the light-chain genes. Phage DNA is islolated from each library, and the heavy- and light-chain sequences are ligated together and packaged to form a combinatorial library. Each phage contains a random pair of heavy- and light-chain cDNAs and upon infection of *E. coli* directs the expression of the antibody chains in infected cells. To identify an antibody that recognizes the antigen of interest, the phage library is plated, and the antibody molecules present in the plaques are transferred to filters. The filters are incubated with radioactively labeled antigen and then washed to remove excess unbound ligand. A radioactive spot on the autoradiogram identifies a plaque that contains an antibody that binds the antigen. Cloning and expression vectors that are useful for producing a human immunoglobulin phage library can be obtained, for example, from STRATAGENE Cloning Systems (La Jolla, CA).

A similar strategy can be employed to obtain high-affinity scFv. See, e.g., Vaughn et al., Nat. Biotechnol., 14: 309-314 (1996). An scFv library with a large repertoire can be constructed by isolating V-genes from non-immunized human donors using PCR primers corresponding to all known V_{H}, V_{κ} and V_{λ} gene families. Following amplification, the V_{κ} and V_{λ} pools are combined to form one pool. These fragments are ligated into a phagemid vector. The scFv linker, (Gly₄, Ser)₃, is then ligated into the phagemid upstream of the V_{L} fragment. The V_{H} and linker-V_{L} fragments are amplified and assembled on the J_{H} region. The resulting V_{H}-linker-V_{L} fragments are ligated into a phagemid vector. The phagemid library can be panned using filters, as described above, or using immunotubes (Nunc; Maxisorp). Similar results can be achieved by constructing a combinatorial immunoglobulin library from lymphocytes or spleen cells of immunized rabbits and by expressing the scFv constructs in *P. pastoris.* See, e.g., Ridder et al., Biotechnology, 13: 255-260 (1995). Additionally, following isolation of an appropriate scFv, antibody fragments with higher binding affinities and slower dissociation rates can be obtained through affinity maturation processes such as CDR3 mutagenesis and chain shuffling. See, e.g., Jackson et al., Br. J. Cancer, 78: 181-188 (1998); Osbourn et al., Immunotechnology, 2: 181-196 (1996).

A variety of recombinant methods can be used to produce bi-specific antibodies and antibody fragments. For example, bi-specific antibodies and antibody fragments can be produced in the milk of transgenic livestock. See, e.g., Colman, A., Biochem. Soc. Symp., 63: 141-147, 1998; U.S. Patent No. 5,827,690. Two DNA constructs are prepared which contain, respectively, DNA segments encoding paired immunoglobulin heavy and light chains. The fragments are cloned into expression vectors which contain a promoter sequence that is preferentially expressed in mammary epithelial cells. Examples include, but are not limited to, promoters from rabbit, cow and sheep casein genes, the cow α-lactoglobulin gene, the sheep β-lactoglobulin gene and the mouse whey acid protein gene. Preferably, the inserted fragment is flanked on its 3' side by cognate genomic sequences from a mammary-specific gene. This provides a polyadenylation site and transcript-stabilizing sequences. The expression cassettes are coinjected into the pronuclei of fertilized, mammalian eggs, which are then implanted into the uterus of a recipient female and allowed to gestate. After birth, the progeny are screened for the presence of both transgenes by Southern analysis. In order for the antibody to be present, both heavy and light chain genes must be expressed concurrently in the same cell. Milk from transgenic females is analyzed for the presence and functionality of the antibody or antibody fragment using standard immunological methods known in the art. The antibody can be purified from the milk using standard methods known in the art.

A chimeric Ab is constructed by ligating the cDNA fragment encoding the mouse light variable and heavy variable domains to fragment encoding the C domains from a human antibody. Because the C domains do not contribute to antigen binding, the chimeric antibody will retain the same antigen specificity as the original mouse Ab but will be closer to human antibodies in sequence. Chimeric Abs still contain some mouse sequences, however, and may still be immunogenic. A humanized Ab contains only those mouse amino acids necessary to recognize the antigen. This product is constructed by building into a human antibody framework the amino acids from mouse complementarity determining regions.

### VII. General Methods for Design and Expression of Mutant Bi-Specific Antibodies

Various mutagenesis techniques may be used to construct the mutant bsAb of the present invention. A person of ordinary skill in the art is well acquainted with such techniques. For example, an expression vector for the mutant bsAb may be obtained by constructing a mutated HC fragment, subcloning this fragment into the expression vector for the parent bsAb to replace the corresponding wild type fragment, and transfecting a host cell with the vector.

In order to obtain an expression vector for the parent bsAb, a person of ordinary skill in the art can use techniques readily available. Some of these techniques are disclosed in WO 1999/66951 Briefly, in order to construct an expression vector of a parent bsAb, such as hMN14IgG-(734 scFv)₂, the gene segment encoding a single chain 734 Fv (734scFv) may be constructed. The 734scFv segment may be linked to the 3'-end of human gamma-chain gene through a DNA fragment coding for a short flexible linker (sL) (Coloma & Morrison 1997 p.787/id) resulting in a fusion gene sequence for C_{H}1-Hinge-C_{H}2-C_{H}3-sL-734scFv (C_{H}-scFv). The C_{H}-scFv fusion gene segment can then be linked to the sequence for hMN-14 V_{H} in an expression vector, hMN14pdHL2, which also contained hMN-14 light chain gene segment, as well as a *dhfr* gene for selection of transfectants and subsequent amplification of the transfected sequences (Dorai & Moore 1987 p. 815/id and Gillies, Lo et al. 1989 p. 131/id). The vector encoding hMN14IgG-(734scFv)₂ (bsAb2pdHL2) may be transfected into Sp2/0 myeloma cells for expression of the fusion bsAb. The bsAb, hMN14IgG-(734scFv)₂, can be purified from culture supernatants by affinity chromatography and analyzed by SDS-PAGE. To evaluate the immunoreactivities of the different biding moieties within a parent or mutant bsAb, competitive ELISA binding assays may be performed.

A bsAbs of IgG-scFv with other specificities and the respective mutant bsAbs can be generated by substitution of only the variable region sequences of the IgG and/or the scFv with those of other Abs. The CDR grafted mutant bsAb can be generated by substitution of only the variable region sequences of the IgG or scFv with those of the CDR grafted Abs. Typically, this "CDR-grafting" technology has been applied to the generation of recombinant, pharmaceutical antibodies consisting of murine CDRs, human variable region frameworks and human constant regions (eg Riechmann, L. et al, (1988) Nature, 332, 323-327). Such "reshaped" or "humanized" antibodies have less murine content than chimeric antibodies and retain the human constant regions necessary for the stimulation of human Fc dependent effector functions. In consequence, CDR grafted antibodies are less likely than chimeric antibodies to evoke a HAMA response when administered to humans, their half-life in circulation should approach that of natural human antibodies and their diagnostic and therapeutic value is enhanced.

In practice, for the generation of efficacious humanized antibodies retaining the specificity of the original murine antibody, it is not usually sufficient simply to substitute CDRs. In addition there is a requirement for the inclusion of a small number of critical murine antibody residues in the human variable region. The identity of these residues depends on the structure of both the original murine antibody and the acceptor human antibody. British Patent Application Number 9019812.8 discloses a method for identifying a minimal number of substitutions of foreign residues sufficient to promote efficacious antigen binding. In one embodiment of the present invention, the Fvs and scFvs of the mutant fusion protein are CDR-grafted murine Fvs and scFvs. In another embodiment of the present invention, the Fvs and scFvs of the mutant fusion protein are humanized. In one embodiment, the Fvs are derived from and the scFvs are 734scFv. In a preferred embodiment of the present invention, the mutant fusion protein is hMN-14IgG^{125A}-(734scFv)₂.

### VIII. Methods of Administration Mutant bsAbs

The present invention contemplates the use of the inventive bispecific antibodies and targetable constructs in treating and/or imaging normal tissue and organs using the methods described in U.S. Patent Nos. 6,126,916; 6,077,499; 6,010,680; 5,776.095; 5,776,094; 5,776,093; 5,772,981; 5,753,206; 5,746,996; 5,697,902; 5,328,679; 5,128,119; 5,101,827; and 4,735,210. Additional methods are described in WO 1999/66951 and WO 02/082041. As used herein, the term "tissue" refers to tissues, including but not limited to, tissues from the ovary, thymus, parathyroid or spleen. Exemplary diseases and conditions that can be treated with the mutant bsAb of the present invention are immune dysregulation disease, an autoimmune disease, organ graft rejection or graft vs. host disease. Immunothereapy of autoimmune disorders using antibodies which target B-cells is described in WO 00/74718 m which claims priority to U.S. Provisional Application 60/138,284. Exemplary autoimmune diseases are acute idiopathic thrombocytopenic purpura, chronic idiopathic thrombocytopenic purpura, dermatomyositis, Sydenham's chorea, myasthenia gravis, systemic lupus erythematosus, lupus nephritis, rheumatic fever, polyglandular syndromes, bullous pemphigoid, diabetes mellitus, Henoch-Schonlein purpura, poststreptococcalnephritis, erythema nodosurn, Takayasu's arteritis, Addison's disease, rheumatoid arthritis, multiple sclerosis, sarcoidosis, ulcerative colitis, erythema multiforme, IgA nephropathy,polyarteritis nodosa, ankylosing spondylitis. Goodpasture's syndrome, thromboangitisubiterans, Sjogren's syndrome, primary biliary cirrhosis, Hashimoto's thyroiditis, thyrotoxicosis, scleroderma, chronic active hepatitis, polymyositis/dermatomyositis ,polychondritis, parnphigus vulgaris, Wegener's granulomatosis, membranous nephropathy, amyotrophic lateral sclerosis, tabes dorsalis, giant cell arteritis/polymyalgia, perniciousanemia, rapidly progressive glomerulonephritis and fibrosing alveolitis.

The mutant bsAb of the present invention may be used in a pretargeting method as the primary targeting species. In pretargeting methods, the mutant bsAb is administered. Once sufficient accretion of the primary targeting species is achieved, a targetable construct is administered. The targetable construct comprises a binding site which recognizes the available binding site of the primary targeting species and a diagnostic or therapeutic agent. Exemplary targetable constructs are described above. The doses and timing of the reagents can be readily worked out by a skilled artisan, and are dependent on the specific nature of the reagents employed. A pretargeting method may be performed with or without the use of a clearing agent.

After sufficient time has passed for the bsAb to target to the diseased tissue, the diagnostic agent is administered. Subsequent to administration of the diagnostic agent, imaging can be performed. Tumors can be detected in body cavities by means of directly or indirectly viewing various structures to which light of the appropriate wavelength is delivered and then collected. Lesions at any body site can be viewed so long as nonionizing radiation can be delivered and recaptured from these structures. For example, PET which is a high resolution, non-invasive, imaging technique can be used with the inventive antibodies for the visualization of human disease. In PET, 511 keV gamma photons produced during positron annihilation decay are detected when using F-18 as the positron-emitter.

The invention generally contemplates the use of diagnostic agents which emit 25-600 keV gamma particles and/or positrons. Examples of such agents include, but are not limited to ¹⁸F, ⁵²Fe,⁶²Cu, ⁶⁴Cu, ⁶⁷Cu, ⁶⁷Ga, ⁶⁸Ga, ⁸⁶Y, ⁸⁹Zr, ^{94m}Tc, ⁹⁴Tc, ^{99m}Tc, ¹¹¹In, ¹²³I, ²⁴¹I, ¹²⁵I, ¹³¹I, ¹⁵⁴⁻¹⁵⁸Gd and ¹⁷⁵Lu.

the use of Detection with intraoperative/endoscopic probes is also contemplated in the use of a mutant bsAb of the present invention and a targetable construct which is a peptide labeled with 1-125. Such methods are disclosed in U.S. Patents 5,716,595 and 6,096,289.

The present mutant bsAb can be used in a method of photodynamic therapy (PDT) as discussed in U.S. Patent Nos. 6,096,289; 4,331,647; 4,818,709; 4,348,376; 4,361,544; 4,444,744; 5,851,527.

In PDT, a photosensitizer, e. g. , a hematoporphyrin derivative such as dihematoporphyrin ether, is administered to a subject. Anti-tumor activity is initiated by the use of light, e.g., 630 nm. Alternate photosensitizers can be utilized, including those useful at longer wavelengths, where skin is less photosensitized by the sun. Examples of such photosensitizers include, but are not limited to, benzoporphyrin monoacid ring A (BPD-MA), tin etiopurpurin (SnET2), sulfonated aluminum phthalocyanine (AlSPc) and lutetium texaphyrin (Lutex).

Additionally, in PDT, a diagnostic agent is injected, for example, systemically, and laser-induced fluorescence can be used by endoscopes to detect sites of cancer which have accreted the light-activated agent. For example, this has been applied to fluorescence bronchoscopic disclosure of early lung tumors. Doiron et al. Chest 76:32 (1979). In another example, the antibodies and antibody fragments can be used in single photon emission. For example, a Tc-99m-labeled diagnostic agent can be administered to a subject following administration of the inventive antibodies or antibody fragments. The subject is then scanned with a gamma camera which produces single-photon emission computed tomographic images and defines the lesion or tumor site.

Therapeutically useful immunoconjugates can be obtained by conjugating photoactive agents or dyes to an antibody composite. Fluorescent and other chromogens, or dyes, such as porphyrins sensitive to visible light, have been used to detect and to treat lesions by directing the suitable light to the lesion. In therapy, this has been termed photoradiation, phototherapy, or photodynamic therapy (Jori et al. (eds.), Photodynamic Therapy of Tumors and Other Diseases (Libreria Progetto 1985); van den Bergh, Chem. Britain 22:430 (1986)). Moreover, monoclonal antibodies have been coupled with photoactivated dyes for achieving phototherapy. Mew et al., J. Immunol. 130:1473 (1983); *idem.,* Cancer Res. 45:4380 (1985); Oseroff et al., Proc. Natl. Acad. Sci. USA 83:8744 (1986); *idem.,* Photochem. Photobiol. 46:83 (1987); Hasan et al., Prog. Clin. Biol. Res. 288:471 (1989); Tatsuta et al., Lasers Surg. Med. 9:422 (1989); Pelegrin et al., Cancer 67:2529 (1991). However, these earlier studies did not include use of endoscopic therapy applications, especially with the use of antibody fragments or subfragments. Thus, the present invention contemplates the therapeutic use of immunoconjugates comprising photoactive agents or dyes.

The linker moiety may also be conjugated to an enzyme capable of activating a prodrug at the target site or improving the efficacy of a normal therapeutic by controlling the body's detoxification pathways. Following administration of the bsAb, an enzyme conjugated to the linker moiety, a low MW hapten recognized by the second arm of the bsAb (the scFv component), is administered. After the enzyme is pretargeted to the target site, a cytotoxic drug is injected, which is known to act at the target site. The drug may be one which is detoxified by the mammal's ordinary detoxification processes. For example, the drug may be converted into the potentially less toxic glucuronide in the liver. The detoxified intermediate can then be reconverted to its more toxic form by the pretargeted enzyme at the target site. Alternatively, an administered prodrug can be converted to an active drug by the pretargeted enzyme. The pretargeted enzyme improves the efficacy of the treatment by recycling the detoxified drug. This approach can be adopted for use with any enzyme-drug pair.

Certain cytotoxic drugs that are useful for anticancer therapy are relatively insoluble in serum. Some are also quite toxic in an unconjugated form, and their toxicity is considerably reduced by conversion to prodrugs. Conversion of a poorly soluble drug to a more soluble conjugate, e.g., a glucuronide, an ester of a hydrophilic acid or an amide of a hydrophilic amine, will improve its solubility in the aqueous phase of serum and its ability to pass through venous, arterial or capillary cell walls and to reach the interstitial fluid bathing the tumor. Cleavage of the prodrug deposits the less soluble drug at the target site. Many examples of such prodrug-to-drug conversions are disclosed in Hansen U.S. Patent No. 5,851,527.

Conversion of certain toxic substances such as aromatic or alicyclic alcohols, thiols, phenols and amines to glucuronides in the liver is the body's method of detoxifying them and making them more easily excreted in the urine. One type of antitumor drug that can be converted to such a substrate is epirubicin, a 4-epimer of doxorubicin (Adriamycin), which is an anthracycline glycoside and has been shown to be a substrate for human beta-D-glucuronidase See, e.g., Arcamone Cancer Res. 45:5995 (1985). Other analogues with fewer polar groups are expected to be more lipophilic and show greater promise for such an approach. Other drugs or toxins with aromatic or alicyclic alcohol, thiol or amine groups are candidates for such conjugate formation. These drugs, or other prodrug forms thereof, are suitable candidates for the site-specific enhancement methods of the present invention.

The prodrug CPT- 11 (irinotecan) is converted *in vivo* by carboxylesterase to the active metabolite SN-38. One application of the invention, therefore, is to use a bsAb targeted against a tumor and a hapten (*e.g*. di-DTPA) followed by injection of a di-DTPA-carboxylesterase conjugate. Once a suitable tumor-to-background localization ratio has been achieved, the CPT-11 is given and the tumor-localized carboxylesterase serves to convert CPT-11 to SN-38 at the tumor. Due to its poor solubility, the active SN-38 will remain in the vicinity of the tumor and, consequently, will exert an effect on adjacent tumor cells that are negative for the antigen being targeted. This is a further advantage of the method. Modified forms of carboxylesterases have been described and are within the scope of the invention. See, e.g., Potter et al., Cancer Res. 58:2646-2651 (1998) and Potter et al., Cancer Res. 58:3627-3632 (1998).

Etoposide is a widely used cancer drug that is detoxified to a major extent by formation of its glucuronide and is within the scope of the invention. See, e.g., Hande et al. Cancer Res. 48:1829-1834 (1988). Glucuronide conjugates can be prepared from cytotoxic drugs and can be injected as therapeutics for tumors pre-targeted with mAb-glucuronidase conjugates. See, e.g., Wang et al. Cancer Res. 52:4484-4491 (1992). Accordingly, such conjugates also can be used with the pre-targeting approach described here. Similarly, designed prodrugs based on derivatives of daunomycin and doxorubicin have been described for use with carboxylesterases and glucuronidases. See, e.g., Bakina et al. J. Med Chem. 40:4013-4018 (1997). Other examples of prodrug/enzyme pairs that can be used within the present invention include, but are not limited to, glucuronide, prodrugs of hydroxy derivatives of phenol mustards and beta-glucuronidase; phenol mustards or CPT-11 and carboxypeptidase; methotrexate-substituted alpha-amino acids and carboxypeptidase A; penicillin or cephalosporin conjugates of drugs such as 6-mercaptopurine and doxorubicin and beta-lactamase; etoposide phosphate and alkaline phosphatase.

The enzyme capable of activating a prodrug at the target site or improving the efficacy of a normal therapeutic by controlling the body's detoxification pathways may alternatively be conjugated to the hapten. The enzyme-hapten conjugate is administered to the subject following administration of the pre-targeting bsAb and is directed to the target site. After the enzyme is localized at the target site, a cytotoxic drug is injected, which is known to act at the target site, or a prodrug form thereof which is converted to the drug *in situ* by the pretargeted enzyme. As discussed above, the drug is one which is detoxified to form an intermediate of lower toxicity, most commonly a glucuronide, using the mammal's ordinary detoxification processes. The detoxified intermediate, *e*.*g*., the glucuronide, is reconverted to its more toxic form by the pretargeted enzyme and thus has enhanced cytotoxicity at the target site. This results in a recycling of the drug. Similarly, an administered prodrug can be converted to an active drug through normal biological processess. The pretargeted enzyme improves the efficacy of the treatment by recycling the detoxified drug. This approach can be adopted for use with any enzyme-drug pair.

The invention further contemplates the use of the inventive bsAb and the diagnostic agent(s) in the context of Boron Neutron Capture Therapy (BNCT) protocols. BNCT is a binary system designed to deliver ionizing radiation to tumor cells by neutron irradiation of tumor-localized ¹⁰B atoms. BNCT is based on the nuclear reaction which occurs when a stable isotope, isotopically enriched ¹⁰B (present in 19.8% natural abundance), is irradiated with thermal neutrons to produce an alpha particle and a ⁷Li nucleus. These particles have a path length of about one cell diameter, resulting in high linear energy transfer. Just a few of the short-range 7 MeV alpha particles produced in this nuclear redaction are sufficient to target the cell nucleus and destroy it. Success with BNCT of cancer requires methods for localizing a high concentration of ¹⁰B at tumor sites, while leaving non-target organs essentially boron-free. Compositions and methods for treating tumors in subjects using pre-targeting bsAb for BNCT are described in WO 00/33874 and can easily be modified for the purposes of the present invention.

It should also be noted that scFv component of the mutant bsAb of the present invention may also be specific to an enzyme.

A clearing agent may be used which is given between doses of the mutant bsAb and the targetable construct. The present inventors have discovered that a clearing agent of novel mechanistic action may be used with the invention, namely a glycosylated anti-idiotypic Fab' fragment targeted against the disease targeting arm(s) of the bsAb. Anti-CEA (MN 14 Ab) x anti-peptide bsAb is given and allowed to accrete in disease targets to its maximum extent. To clear residual bsAb, an anti-idiotypic Ab to MN-14, termed WI2, is given, preferably as a glycosylated Fab' fragment. The clearing agent binds to the bsAb in a monovalent manner, while its appended glycosyl residues direct the entire complex to the liver, where rapid metabolism takes place. Then the therapeutic which is associated with the linker moiety is given to the subject. The WI2 Ab to the MN-14 arm of the bsAb has a high affinity and the clearance mechanism differs from other disclosed mechanisms (see Goodwin *et al., ibid*)*,* as it does not involve cross-linking, because the WI2-Fab' is a monovalent moiety.

The present mutant bsAb can also be used in a method of ultrasound imaging. An ultrasound enhancement agent, such as a contrast agent, may be attached to a targetable construct, such as a bivalent DTPA peptide. By way of non-limiting example, an enhancement agent such as a liposome, preferably a gas-filled liposome may be used. In this method, the mutant bsAb would be administered first, followed by administration of the liposome-targetable construct complex. See Maresca, G. et al., Eur J. Radiol. Suppl. 2 S171-178 (1998); Demos, Sm. Et al. J. Drug Target 5 507-518 (1998); and Unger, E. et al., Am J. Cardiol. 81 58G-61G (1998).

The mutant bispecific antibody may be administered as one component of a multicomponent treatment regimen. The mutant bispecific antibody may be administered before, during or after the administration of at least one therapeutic agent used to treat a disease or condition.

The use of an exemplary mutant bsAb in a pretargeting method, compared to the use of a parent bsAb in a pretargeting method is illustrated in Example 2. The data illustrates the accelerated rate of clearance of a mutant bsAb of the present invention as compared to the parent bsAb. Additionally, the data illustrates that a much larger amount of targetable construct is trapped in the blood when the parent bsAb is used as compared to when the mutant bsAb is used.

Figures 5 and 6 show data for pretargeting methods involving the parent bsAb, ¹²⁵I-hMN-14IgG-(734scFv)₂. Figure 7 shows data for pretargeting methods involving the mutant bsAb, ¹²⁵I-hMN-14IgG^{1253A}-(734scFv)₂. The ¹²⁵I-label allows for a determination of the amount of bsAb present in different regions of the body. A comparison of the data in Figures 5 and 7 shows that the mutant bsAb cleared the body faster than the parent bsAb. For example, after pretargeting with parent bsAb for 4 days (Figure 5), and 3 hours post injection of IMP-192, the %ID/g for tumor and blood was 19.21 ± 7.318 and 3.73 ± 0.75, respectively. In contrast, after pretargeting with mutant bsAb for 4 days (Figure 5), and 3 hours post injection of IMP-192, the %ID/g for tumor and blood was 2.42 ± 0.78 and 0.07 ± 0.01, respectively.

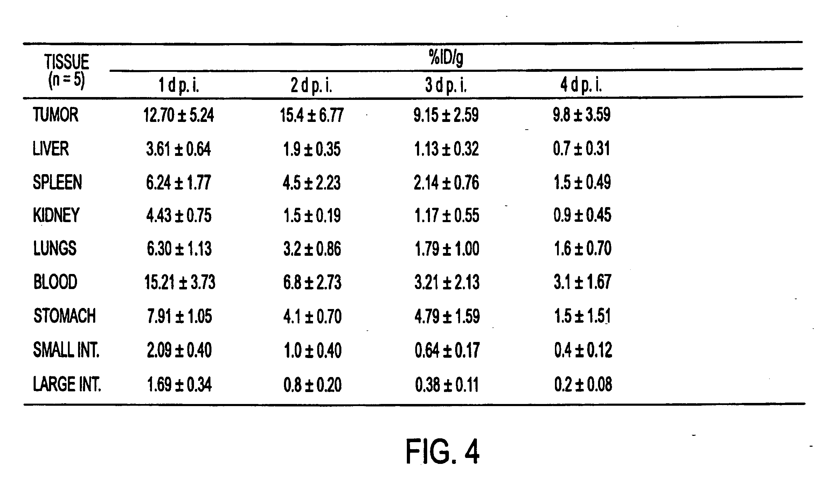

A comparison of the tumor-to-blood ratios of ¹²⁵I in Figures 5 and 7 (see entry under "Blood" in Figures 5 and 7) demonstrates that a higher signal-to-background can be achieved with the mutant bsAb. Even after 6 days of pretargeting with parent bsAb (see Figure 4), the tumor-to-blood ratio is much less than after 4 days of pretargeting with mutant bsAb.

The ^{99m}Tc-label allows for a determination of the amount of targetable construct present in different regions of the body. A comparison of the %ID/g of IMP-192 (^{99m}Tc-labeled targetable construct) shows that the tumor-to-blood ratio is much greater for the pretargeting methods with mutant bsAb. This result illustrates that less targetable construct is trapped in the blood in pretargeting methods involving a mutant bsAb. When the parent bsAb is used (see Figures 5 and 6) the ^{99m}Tc-labeled targetable construct is trapped in the blood, rather than appearing at the tumor site. Therefore, low tumor-to-blood ratios are observed. For example, the tumor-to-blood ratio of ^{99m}Tc-labeled targetable construct is shown in Figure 5 (parent bsAb) in the left hand side, under "Blood". Three hours post injection, the tumor-to-blood ratio is 0.24 ± 0.05. In contrast, Figure 5 (mutant bsAb) shows the tumor to blood ratio three hours post injection is 3.52 ± 1.45.

### IX. Other Applications

The present invention encompasses the use of the mutant bsAb and a therapeutic agent associated with the linker moieties discussed above in intraoperative, intravascular, and endoscopic tumor and lesion detection, biopsy and therapy as described in U.S. Patent Nos. 5,716,595 and 6,096,289.

The mutant bsAb of the present invention can be employed not only for therapeutic or imaging purposes, but also as aids in performing research *in vitro.* For example, the bsAbs of the present invention can be used *in vitro* to ascertain if a targetable construct can form a stable complex with one or more bsAbs. Such an assay would aid the skilled artisan in identifying targetable constructs which form stable complexes with bsAbs. This would, in turn, allow the skilled artisan to identify targetable constructs which are likely to be superior as therapeutic and/or imaging agents.

The assay is advantageously performed by combining the targetable construct in question with at least two molar equivalents of a mutant bsAb. Following incubation, the mixture is analyzed by size-exclusion HPLC to determine whether or not the construct has bound to the bsAb. Alternatively, the assay is performed using standard combinatorial methods wherein solutions of various bsAbs are deposited in a standard 96 well plate. To each well, is added solutions of targetable construct(s). Following incubation and analysis, one can readily determine which construct(s) bind(s) best to which bsAb(s).

It should be understood that the order of addition of the mutant bsAb to the targetable construct is not crucial; that is, the mutant bsAb may be added to the construct and vice versa. Likewise, neither the mutant bsAb nor the construct needs to be in solution; that is, they may be added either in solution or neat, whichever is most convenient. Lastly, the method of analysis for binding is not crucial as long as binding is established. Thus, one may analyze for binding using standard analytical methods including, but not limited to, FABMS, high-field NMR or other appropriate method in conjunction with, or in place of, size-exclusion HPLC.

### X. Examples

### Materials And Methods

### Designing and Construction of 734scFv

734scFv was designed to have the configuration of sL-Vλ-L-V_{H}, where sL is a short flexible linker, Gly-Gly-Gly-Ser (Coloma & Morrison, Nat. Biotechnol. 15:159-163 (1997)), serving as the linkage between hMN-14 IgG heavy chain and 734scFv, and L is a long linker between the Vλ and V_{H} of 734 composed of three repeats of Gly-Gly-Gly-Gly-Ser, (Huston, Levinson, et al. PNAS 85:5879-5883 (1988)). Primer pairs 734V_{L}scFv5'(Cys)/734VLscFv3' and 734V_{H}scFv5'/734V_{H}scFv3'(SacI) were used to amplify respective V_{I} and V_{H} sequences of 734. The resulting DNA products were assembled into 734scFv gene by restriction enzyme digestion and ligation and the sequence was confirmed by DNA sequencing.

| | |
|---|---|
| 734VLscFv5'(Cys) | |
| 734VLscFv3' | |
| 734V_{H}scFv5' | 5'-CC GGA GGC GGT GGG AGT GAG GTG AAA CTG CAG GAG-3' |
| 734V_{H}scFv3'(SacI) | |

### Construction of the expression vector for hMN-14IgG-(734scFv)₂

To link 734scFv to the C-terminal end of human heavy constant chain (HC), a new pair of primers, 734scFv2-5' and 734scFv-3', was synthesized and used to amplify the DNA encoding 734scFv. The primer 734scFv2-5' provided the correct sequence for inframe linking 734scFv to the C-terminal end of human HC. The resulting DNA fragment was ligated to human HC sequence, forming a construct encoding HC-734scFv. The DNA fragment encoding normal human HC in the expression vector for hMN-14, hMN-14pdHL2, was then replaced by the HC-734scFv fragment, resulting in the expression vector for the fusion construct, hMN-14IgG-(734scFv)₂pdHL2.

| | |
|---|---|
| 734scFv2-5' | 5'-TCC CCG GGT AAA GGT GGC GGT TCA CAG CTG-3' |
| 734scFv-3' | 5'-GAG CTC GGC CGT CGC AC-3' |

### Construction of the mutant fusion bsAb, hMN-14IgG^{(1253A)}-(734scFv)₂

Isoleucine 253 is located in the C_{H}2 domain of human HC chain. To introduce the I253A mutation into hMN-14IgG-(734scFv)₂, plasmid vector C_{H}1kbpKS, containing an insert DNA fragment encoding C_{H}1 and partial C_{H}2 domains was used in oligonucleotide directed site-specific mutagenesis. An oligonucleotide I253AC_{H}2, which converts the wild type sequence KDTLM²⁵³ISRTPE in the C_{H}2 to KDTLM²³⁵ASRTPE, was designed and synthesized as the mutagenic primer. The mutagenisis was accomplished by using the Sculptor IVM system (Amersham, Arlington Heights, IL) according to the manufacturer's specifications. After the sequence had been verified by dideoxy DNA sequencing, the mutated HC fragment was subcloned into hMN-14IgG-(734scFv)₂pdHL2 to replace the corresponding wild type fragment, resulting in the expression vector for the mutant fusion bsAb, hMN-14IgG^{(I253A)}-( 734scFv)₂pdHL2.

| | |
|---|---|
| I253AC_{H}2 | 5'-AAG GAC ACC CTC ATG GCT AGC CGG ACC CCT GAG-3' |

### Expression and production of bsAbs

The expression vectors were transfected into Sp2/0 cells by electroporation 2-5x10⁶ cells were transfected using ⁻30 µg of SalI linearized DNA and plated into 96-well cell culture plates. After 2 days, methotrexate (MTX) at a final concentration of 0.025-0.075 µM was added into the cell culture medium for the selection of transfectants. MTX-resistant colonies emerged in 2-3 weeks and were screened by ELISA for secretion of human IgG. Briefly, cell culture supernatants from the surviving colonies were incubated in microwells of ELISA plate coated with goat anti-human IgG F(ab')₂ specific antibody for 1 h. A peroxidase-conjugated goat anti-human IgG Fc fragment specific antibody was then added and incubated in the wells for 1 h. The presence of human IgG in the supernatant was revealed by addition of the substrate solution containing 0.4 mg/ml of o-phenylenediamine dihydrochloride and 0.0125% H₂O₂. From the positive clones, the best Ab-producers were determined, selected and further expanded. hMN-14IgG-(734scFv)₂ and hMN-14IgG^{(I253A)}-(734scFv)₂ were purified from cell culture supernatant by affinity chromatography on either Protein A or DTPA column.

### Synthesis of Ac-Lys(DTPA)-Tyr-Lys(DTPA)-Lys(TscG-Cys-)-NH2 (IMP 192):

The first amino acid, Aloc-Lys(Fmoc)-OH was attached to 0.21 mmol Rink amide resin on the peptide synthesizer followed by the addition of the Tc-99m ligand binding residues Fmoc-Cys(Trt)-OH and TscG to the side chain of the lysine using standard Fmoc automated synthesis protocols to form the following peptide: Aloc-Lys(TscG-Cys(Trt)-rink resin. The Aloc group was then removed by treatment of the resin with 8 mL of a solution containing 100 mg Pd[P(Ph)₃]₄ dissolved in 10 mL CH₂Cl₂, 0.75 mL glacial acetic acid and 2.5 ml diisopropylethyl amine. The resin mixture was then treated with 0.8 ml tributyltin hydride and vortex mixed for 60 min. The peptide synthesis was then continued on the synthesizer to make the following peptide: Lys(Aloc)-Tyr-Lys(Aloc)-Lys(TscG-Cys-)-rink resin. The N-terminus was acetylated by vortex mixing the resin for 60 mm with 8 mL of a solution containing 10 mL DMF, 3 mL acetic anhydride, and 6 mL diisopropylethylamine. The side chain Aloc protecting groups were then removed as described above and the resin treated with piperidine using the standard Fmoc deprotection protocol to remove any acetic acid which may have remained on the resin.

*Activated DTPA and DTPA Addition:* The DTPA, 5 g, was dissolved in 40 mL 1.0 M tetrabutylammonium hydroxide in methanol. The methanol was removed under hi-vacuum to obtain a viscous oil. The oil was dissolved in 50 mL DMF and the volatile solvents were removed under hi-vacuum on the rotary evaporator. The DMF treatment was repeated two more times. The viscous oil was then dissolved in 50 ml DMF and mixed with 5 g HBTU. An 8 ml aliquot of the activated DTPA solution was then added to the resin which was vortex mixed for 14 hr. The DTPA treatment was repeated until the resin gave a negative test for amines using the Kaiser test.

*Cleavage and Purification*: The peptide was then cleaved from the resin by treatment with 8 ml of a solution made from 30 ml TFA, 1 ml triisopropylsilane, and 1 ml ethanedithiol for 60 mm. The crude cleaved peptide was precipitated by pouring into 30 ml ether and was collected by centrifugation. The peptide was then purified by reverse phase HPLC using a 4 x 30 cm Waters preparative C-18 Delta-Pak column (15 *µ*m, 100Å). The HPLC fractions were collected and lyophilized to obtain a fraction which contained the desired product by ESMS (MH ± 1590).

*Kit Formulation*: The peptide was formulated into lyophilized kits which contained 78 *µ*g of the peptide, 0.92 mg non-radioactive InCl₃, 100 *µ*g stannous chloride, 3 mg gentisic acid, and HPCD (10 % on reconstitution).

### Radiolabeling

60 µg of antibody protein was labeled with I-125 using the chloramine-T method (Greenwood, Hunter, et al., Biochem. J. 89 11-123 (1963)) and purified using NAP-5 disalting column (Pharmacia, Piscataway, NJ).

To prepare Tc-99m labeled IMP-192, a kit containing 50 *µ*g IMP-192 was reconstituted with 1.5 ml of a saline solution containing 20 mCi pertechnetate. The reconstituted kit was incubated at room temperature for 10 min and then heated for 15 min in a boiling water bath.

### Example 1: Biodistribution ¹²⁵I-hMN-14IgG^{I253A}-(734scFv)₂ and ¹²⁵I-hMN-14IgG-(734scFv)₂ in human colonic tumor-bearing mice.

### Experimental Procedure

Simple biodistribution patterns of the ¹²⁵I-hMN-14IgG-(734scFv)₂ and ¹²⁵I-hMN-14IgG^{I253A}-(734scFv)₂ were evaluated. Groups of nude female mice bearing GW39 human colonic cancer xenografts received i.v. injections of 20 µg (5 µCi)/mouse of a ¹²⁵I-labeled parent or mutant bsAb. Mice were euthanized at designed postinjection time points and their organs were removed, weighted and counted for I-125 radioactivity.

The GW-39 human colonic tumor cell line was propagated as serial, subcutaneous xenografts in nude mice as described elsewhere (Tu, et al. Tumour Biology 9:212-220 (1988)).

### Results

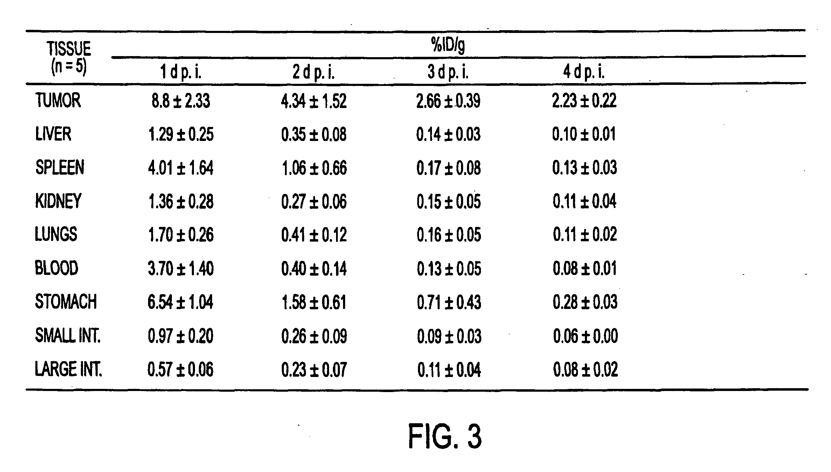

The tumor and normal tissue biodistribution of ¹²⁵I-labeled hMN-14IgG-(734scFv)₂ and hMN-14IgG^{I253A}(734scFv)₂ mutant was examined in human colonic tumor-bearing mice 1, 2, 3 and 4 days postinjection. The results are presented in Figures 3 and 4 wherein data are expressed as a median percentage of injected dose per gram (%ID/g).

The tumor uptake of hMN-14IgG^{I253A}(734scFv)₂ was significantly lower than that of hMN-14IgG-(734scFv)₂. This accelerated rate of clearance of hMN-14IgG^{I253A}(734scFv)₂ is also seen in normal tissues such as liver, spleen, kidney, lungs, stomach, small intestine, large intestine and blood. See Figures 3 and 4. The accelerated clearance of hMN-14IgG^{I253A}(734scFv)₂ produced higher tumor-to-organ ratios for many normal tissues, such as liver, spleen, kidney, lungs, stomach, small intestine, large intestine and blood. Additionally, the tumor-to-blood ratio for the hMN-14IgG^{I253A}(734scFv)₂ mutant increased at a much faster from one to four days postinjection as compared to the tumor/blood ratio for hMN-14IgG-(734scFv)₂.

### Example 2: Pretargeting of ¹²⁵I-hMN-14IgG^{I253A}-(734scFv)₂ and ¹²⁵I-hMN-14IgG-(734scFv)₂ in human colonic tumor-bearing mice

### Experimental Procedure

Pretargeting biodistribution patterns of mutant and parent bsAbs were evaluated. Groups of nude female mice bearing GW39 human colonic cancer xenografts received i.v. injections of 20 µg (5 µCi)/mouse of a ¹²⁵I-labeled mutant or parent bsAb. Following the injection of mutant or parent bsAb, a predetermined clearance time was allowed for bsAb to localize to tumor sites and be removed from circulation. The ^{99m}Tc-labeled divalent DTPA peptide, IMP-192, was then administered i.v. The mice were sacrificed at various time points of postinjection of the peptide and their organs were removed, weighted and counted for both I-125 and Tc-99m radioactivities.

The GW-39 human colonic tumor cell line was propagated as serial, subcutaneous xenografts in nude mice as described elsewhere (Tu, et al. Tumour Biology 9:212-220 (1988)).

### Results

The tumor and normal tissue biodistribution of ¹²⁵I-labeled hMN-14IgG^{I253A}-(734scFv)₂ and ¹²⁵I-labeled hMN-14IgG-(734scFv)₂ was examined in human colonic tumor-bearing mice 3, 6 and 24 hours postinjection of ^{99m}Tc-labeled divalent DTPA peptide, IMP-192. Prior to injection of IMP-192 pretargeting with mutant or parent bsAb was performed for four days. The tumor and normal tissue biodistribution of ¹²⁵I-labeled mutant and parent bsAb are shown in Figures 5-7, wherein data are expressed as a median percentage of injected dose per gram (%ID/g). Additionally, the tumor and normal tissue biodistribution of IMP-192 (^{99m}Tc-labeled divalent DTPA peptide) are shown in Figures 5-7. Accelerated clearance of the mutant bsAb is observed. Additionally, higher tumor-to-blood ratios are observed after pretargeting with mutant bsAb as compared to pretargeting with parent bsAb. It is noted that more DTPA-peptide was trapped in the blood after pretargeting with the parent fusion protein then after pretargeting with the mutant fusion protein.

### Example 3: Binding of In-DTPA containing Peptides to hMN-14IgG^{I253A}-(734scFv)₂

The binding of In-DTPA peptides to the anti-In-DTPA antibody hMN-14IgG^{(I253A)}-(734scFv)₂ was examined by size exclusion HPLC and by affinity blocking studies using the Biacore X:

### Binding Analysis Using HPLC

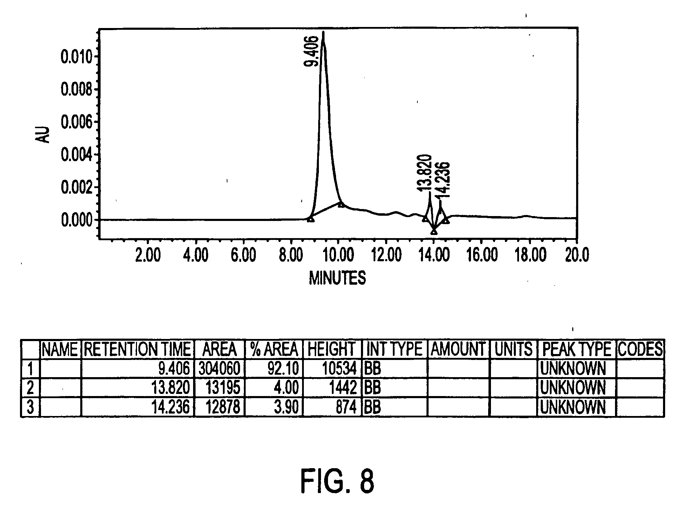

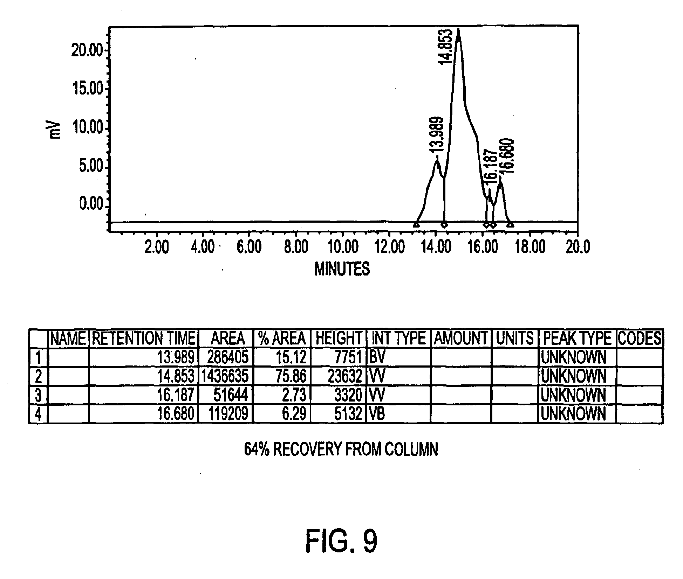

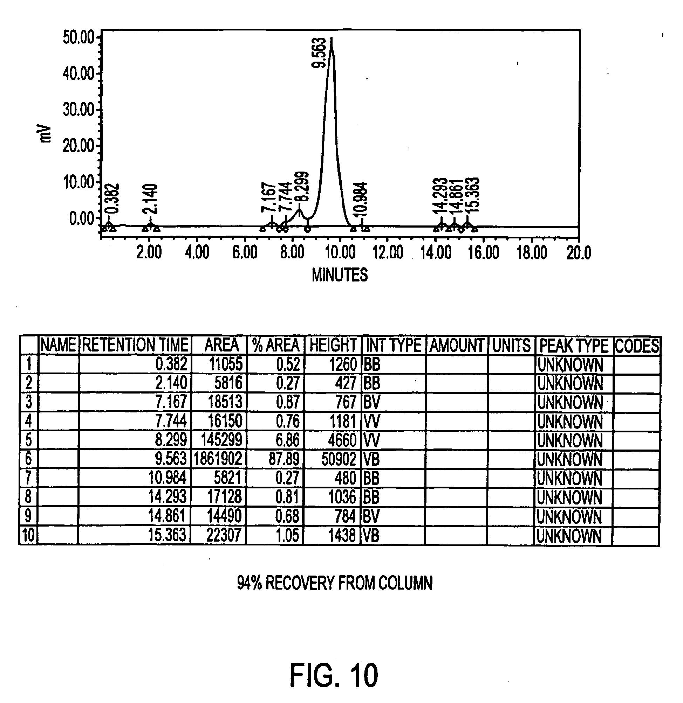

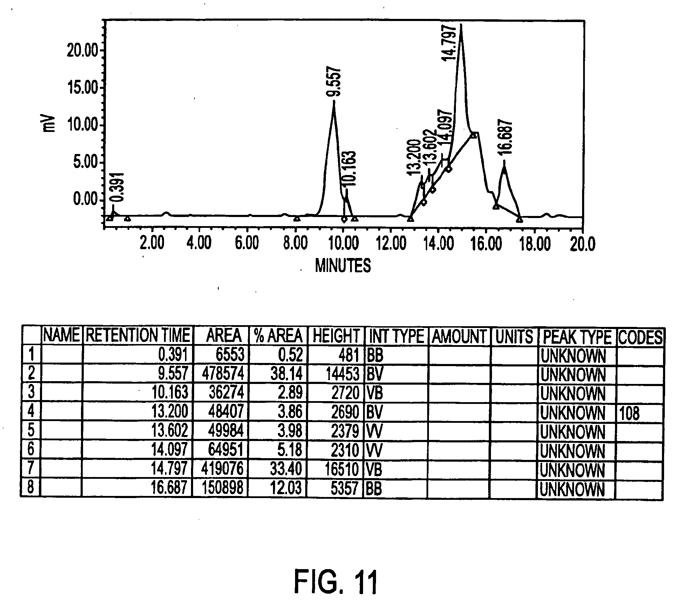

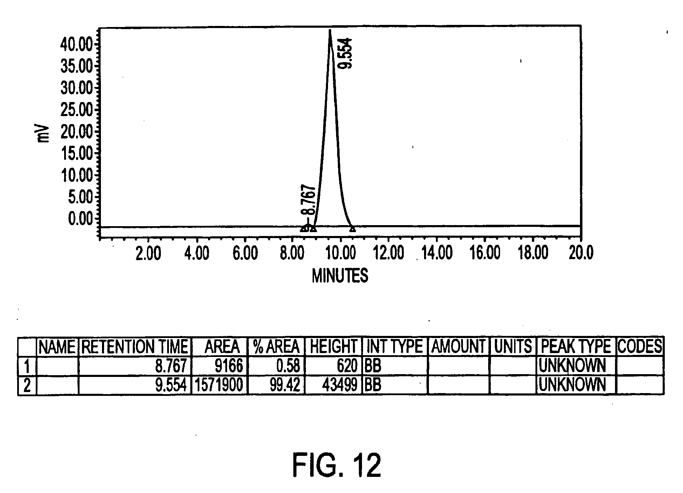

An IMP 192 kit was labeled with Tc-99m 20.9 mCi. Aliquots from the kit were diluted and mixed with hMN-14IgG^{(I253A)}-(734scFv)₂ in the following molar ratios (Peptide/ab) 1:5, 1:1, and 20:1. The peptide/antibody mixtures, the peptide alone and the antibody alone were examined on a Bio-Sil SEC 250 300 mm x 7.8 mm HPLC column elluted at 1 mL/min with 0.2 M phosphate buffer pH 6.8. The HPLC traces (Figures 8-12 show essentially only one peptide/antibody complex is formed. A known standard of hMN-14IgG^{I253A}-(734scFv)₂ eluted from the column at about 9.41 minutes (Figure 8). A known standard of Tc-99m IMP 192 eluted from the column at about 14.85 minutes (Figure 9). When a 1:1 mixture of hMN-14IgG^{I253A}-(734scFv)₂ to Tc-99m IMP 192 were applied to the column, only one peak was observed at about 9.56 minutes (Fig. 10). In contrast, when a 1:5 mixture of hMN-14IgG^{I253A}-(734scFv)₂ to Tc-99m IMP 192 was applied to the column, two major peaks were observed, one at about 9.56 minutes (hMN-14IgG^{I253A}-(734scFv)₂) and the other at about 14.80 minutes (Tc-99m IMP 192) (Fig. 11). When a 20: mixture of hMN-14IgG^{I253A}-(734scFv)₂ to Tc-99m IMP 192 was applied to the column, only one peak was observed at 9.56 minutes (Fig. 12).

### Example 4: Clinical Examples

Example 4A. A patient with a colon polyp has the polyp removed, and it is found to be malignant. CAT scan fails to demonstrate any tumor, but the patient after three months has a rising blood CEA level. The patient is given 10 mg of hMN14-IgG[734-scFv]2 by i.v. infusion. Three days later the patient is given the bivalent peptide IMP 192 labeled with 40 mCi of Tc-99m. The next day the patient undergoes radioscintigraphy, and a single locus of activity is observed in a node close to the site of the resected polph. The node is resected, and patient remains free of disease for the next 10 years.

Example 4B. A patient with colon carcinoma undergoes resection of the primary tumor. Two years later the patient presents with a rising CEA blood level, and CAT scan demonstrates multiple small metastasis in the liver, which cannot be resected. The patient is given 100 mg of hMN14-IgG[734-scFv]2 by i.v. infusion. After 3 days the patient if given the bivalent-DTPA peptide, IMP 156, labeled with 160 mCi of I-131 by i.v. infusion. The CEA blood level slowly drops into the normal range. CAT scan demonstrates resolution of several of the metastasis, and the remaining lesions fail to grow for the next 9 months.

## Claims

1. A mutant bispecific antibody, comprising at least:
(a) a human hinge-Fc constant region from IgG;
(b) two scFvs that each specifically binds to a binding site on a bivalent targetable construct such that a one to one binding interaction is obtained between the mutant bispecific antibody and the targetable construct; and
(c) two Fvs,
wherein said constant region contains one or more amino acid mutations in the C_{H}2 domain, wherein said hinge constant region contains a mutation in which isoleucine at position 253 is replaced with alanine, or an amino acid other than leucine, and wherein the amino acid replacement enhances blood clearance equal to, or greater than replacement with alanine.

2. The mutant bispecific antibody of claim 1, wherein said scFvs and said Fvs are CDR-grafted murine scFvs and Fvs.

3. The mutant bispecific antibody of claim 1, wherein said scFvs and said Fvs are humanized or human.

4. The mutant bispecific antibody of claim 1, comprising the Fv regions of hMN14-IgG and 734scFv.

5. The mutant bispecific antibody of claim 1, wherein the bivalent targetable construct is conjugated to one or more drugs, toxins, radioisotopes, enzymes or labels.

6. The mutant bispecific antibody of claim 1, wherein said Fvs bind to an epitope on a target cell.

7. The mutant bispecific antibody of claim 6, wherein said Fvs bind to a tumor associated antigen.

8. The mutant bispecific antibody of claim 7, wherein said Fvs bind to CEA (carcinoembryonic antigen).

9. The mutant bispecific antibody of claim 1, wherein said mutant bispecific antibody incorporates the Fv of a class III anti-CEA antibody.

10. The mutant bispecific antibody of claim 1, wherein said mutant bispecific antibody incorporates the scFv of Mab 679.

11. A product comprising:
(A) a mutant bispecific antibody of any of claims 1 to 10;
(B) optionally, a clearing composition; and
(C) a targetable construct comprising a bivalent hapten, wherein both hapten moieties bind to the two scFvs on a single molecule of the mutant bispecific antibody, wherein the targetable construct further comprises a therapeutic cation, and/or one or more chelated or chemically bound therapeutic agents; as a combined preparation for simultaneous, separate or sequential use for treatment or diagnosis of a disease of a subject.

12. The product of claim 11, which further comprises at least one therapeutic agent used to treat the disease

13. The product of claim 11, wherein said targetable construct comprises an enzyme and which further comprises:
a) a prodrug, wherein said enzyme is capable of converting said prodrug to a drug at the target site; or
b) a drug which is capable of being detoxified in said subject to form an intermediate of lower toxicity, wherein said enzyme is capable of reconverting said detoxified intermediate to a toxic form, and, increasing the toxicity of said drug at the target site; or
c) a prodrug which is activated in said subject through natural processes and is subject to detoxification by conversion to an intermediate of lower toxicity, wherein said enzyme is capable of reconverting said detoxified intermediate to a toxic form, and, increasing the toxicity of said drug at the target site.

14. The product of claim 13, wherein said prodrug is selected from the group consisting of epirubicin glucuronide, CPT-11, etoposide glucuronide, daunomicin glucuronide and doxorubicin glucuronide

15. The product of claim 11, wherein the therapeutic agent is selected from the group consisting of ¹⁸F, ⁵²Fe, ⁶²Cu, ⁶⁴Cu, ⁶⁷Cu, ⁶⁷Ga, ⁶⁸Ga, ⁸⁶Y, ⁸⁹Zr, ^{94m}Tc, ⁹⁴Tc, ^{99m}Tc, ¹¹¹In, ¹²³I, ¹²⁴I, ¹²⁵I, ¹³¹I, ¹⁵⁴⁻¹⁵⁸Gd, ¹⁷⁷Lu, ³²P, ¹⁸⁸Re, and ⁹⁰Y or a combination thereof.

16. The product of claim 15, wherein said radioactive isotope is used to perform positron-emission tomography (PET).

17. The product of claim 11, wherein said targetable construct comprises one or more image enhancing agents for use in magnetic resonance imaging (MRI).

18. The product of claim 17, wherein said enhancing agent is selected from the group consisting of Mn, Fe and Gd.

19. The product of claim 11, wherein said targetable construct comprises one or more image enhancing agents for use in ultrasound imaging.

20. The product of claim 19, wherein said targetable construct is a liposome with a bivalent DTPA-peptide covalently attached to the outside surface of the liposome lipid membrane.

21. The product of claim 20, wherein said liposome is gas filled.

22. The product of claim 11, wherein said targetable construct comprises one or more radioactive isotopes useful for killing diseased tissue.

23. The product of claim 22, wherein the energy range of the radioactive isotope is 60 to 700 keV.

24. The product of claim 22, wherein said radioactive isotope is selected from the group consisting of ³²P, ³³P, ⁴⁷Sc, ⁶⁴Cu, ⁶⁷Cu, ⁶⁷Ga, ⁹⁰Y, ¹¹¹Ag, ¹¹¹In, ¹²⁵I, ¹³¹I, ¹⁴²Pr, ¹⁵³Sm, ¹⁶¹Tb, ¹⁶⁶Dy, ¹⁶⁶Ho, ¹⁷⁷Lu, ¹⁸⁶Re, ¹⁸⁸Re, ¹⁸⁹Re, ²¹²Pb, ²¹²Bi, ²¹³Bi, ²¹¹At, ²²³Ra, ²²⁵Ac and a combination thereof.

25. The product of claim 22, wherein said targetable construct comprises ¹⁰B atoms, and said treatment of a disease further comprises the step of irradiating said boron atoms localized at said diseased tissue, thereby effecting BNCT of said diseased tissue.

26. The product of claim 11, wherein said therapeutic agent is a drug, toxin, hormone, enzyme, immunomodulator, chelator, boron compound, photoactive agent, dye, or radioisotope.

27. The product of claim 11, wherein said targetable construct comprises one or more toxins.

28. The product of claim 27, wherein said toxin is selected from the group consisting of ricin, abrin, ribonuclease, DNase 1, Staphylococcal enterotoxin-A, pokeweed antiviral protein, gelonin, diphtherin toxin, Pseudomonas exotoxin, Pseudomonas endotoxin and a combination thereof.

29. The product of claim 11, wherein said targetable construct comprises one or more drugs.

30. The product of claim 29, wherein said drug is selected from the group consisting of nitrogen mustards, ethylenimine derivatives, alkyl sulfonates, nitrosoureas, triazenes, folic acid analogs, anthracyclines, taxanes, COX-2 inhibitors, pyrimidine analogs, purine analogs, antibiotics, enzymes, epipodophyllotoxins, platinum coordination complexes, vinca alkaloids, substituted ureas, methyl hydrazine derivatives, adrenocortical suppressants, antagonists, endostatin, taxols, camptothecins, doxorubicins and their analogs, and a combination thereof.

31. The product of claim 11, wherein the targetable construct comprises one or more agents for photodynamic therapy.

32. The product of claim 31, wherein said agent for photodynamic therapy is a photosensitizer.

33. The product of claim 32, wherein said photosensitizer is selected from the group consisting of benzoporphyrin monoacid ring A (BPD-MA), tin etiopurpurin (SnET2), sulfonated aluminum phthalocyanine (AlSPc) and lutetium texaphyrin (Lutex).

34. The product of claim 11, comprising mutant bispecific antibody of claim 8 wherein said Fvs bind to a marker substance associated with a tumor, and wherein said tumor produces or is associated with antigens selected from the group consisting of colon-specific antigen-p (CSAp), carcinoembryonic antigen (CEA), CD4, CD5, CD8, CD14, CD15, CD19, CD20, CD21, CD22, CD23, CD25, CD33, CD37, CD38, CD40, CD40L, CD46, CD52, CD54, CD66a-e, CD74, CD75, CD80, CD 126, B7, HLA-DR, Ia, Ii, HM1.24, MUC 1, MUC2, MUC3, MUC4,Tag-72, PSMA, EGP-1, EGP-2, PSA, AFP, HCG, HCG-beta, PLAP, PAP, histone, tenascin, VEGF, P1GF, S100, EGFR, insulin-like growth factor, HER2/neu, organotropic hormones, oncogene products, and cytokeratin.

35. The product of claims 11 or 13, wherein the mutant bispecific antibody incorporates the Fv of a Class III anti-CEA antibody.

36. The product of claims 11 or 13, wherein the mutant bispecific antibody incorporates the scFv of Mab 679.

37. The product of claim 11, wherein said disease is an immune dysregulation disease, an autoimmune disease, organ graft rejection, cardiovascular disease, neurological disease or graft vs. host disease.

38. The product of claim 37, wherein said autoimmune disease is selected from the group consisting of acute idiopathic thrombocytopenic purpura, chronic idiopathic thrombocytopenic purpura, dermatomyositis, Sydenham's chorea, myasthenia gravis, systemic lupus erythematosus, lupus nephritis, rheumatic fever, polyglandular syndromes, bullous pemphigoid, diabetes mellitus, Henoch-Schonlein purpura, poststreptococcalnephritis, erythema nodosurn, Takayasu's arteritis, Addison's disease, rheumatoid arthritis, multiple sclerosis, sarcoidosis, ulcerative colitis, erythema multiforme, IgA nephropathy, polyarteritis nodosa, ankylosing spondylitis, Goodpasture's syndrome, thromboangitisubiterans, Sjogren's syndrome, primary biliary cirrhosis, Hashimoto's thyroiditis, thyrotoxicosis, scleroderma, chronic active hepatitis, polymyositis/dermatomyositis, polychondritis, parnphigus vulgaris, Wegener's granulomatosis, membranous nephropathy, amyotrophic lateral sclerosis, tabes dorsalis, giant cell arteritis/polymyalgia, pernicious anemia, rapidly progressive glomerulonephritis and fibrosing alveolitis.

39. The product of claims 11 or 13, wherein said disease is caused by a fungus, virus, parasite or bacterium, and the Fv of the mutant bispecific antibody targets the fungus, virus, parasite, or bacterium.

40. The product of claim 39, wherein said virus is selected from the group consisting of human immunodeficiency virus (HIV), herpes virus, cytomegalovirus, rabies virus, influenza virus, hepatitis B virus, Sendai virus, feline leukemia virus, Reo virus, polio virus, human serum parvo-like virus, simian virus 40, respiratory syncytial virus, mouse mammary tumor virus, Varicella-Zoster virus, Dengue virus, rubella virus, measles virus, adenovirus, human T-cell leukemia viruses, Epstein-Barr virus, murine leukemia virus, mumps virus, vesicular stomatitis virus, Sindbis virus, lymphocytic choriomeningitis virus, wart virus and blue tongue virus.

41. The product of claim 39, wherein said bacterium is selected from the group consisting of Anthrax bacillus, Streptococcus agalactiae, Legionella pneumophilia, Streptococcus pyogenes, Escherichia coli, Neisseria gonorrhoeae, Neisseria meningitidis, Pneumococcus, Hemophilis influenzae B, Treponema pallidum, Lyme disease spirochetes, Pseudomonas aeruginosa, Mycobacterium leprae, Brucella abortus, Mycobacterium tuberculosis and Tetanus toxin.

42. The product of claim 39, wherein said pathogen is a protozoan.

43. The product of claim 42, wherein said protozoan is selected from the group consisting of Plasmodium falciparum, Plasmodium vivax, Toxoplasma gondii, Trypanosoma rangeli, Trypanosoma cruzi, Trypanosoma rhodesiensei, Trypanosoma brucei, Schistosoma mansoni, Schistosoma japanicum, Babesia bovis, Elmeria tenella, Onchocerca volvulus, Leishmania tropica, Trichinella spiralis, Onchocerca volvulus, Theileria parva, Taenia hydatigena, Taenia ovis, Taenia saginata, Echinococcus granulosus and Mesocestoides corti.

44. The product of claim 39, wherein said parasite is a helminth or a malarial parasite.

45. The product of claim 39, wherein said bacterium is mycoplasma.

46. The product of claim 45, wherein said mycoplasina is selected from the group consisting of Mycoplasma arthritidis, M. hyorhinis, M. orale, M. arginini, Acholeplasma laidlawii, M. salivarum, and M. pneumoniae.

47. The product of claim 39, wherein the fungus is selected from the group consisting of Histoplasma capsulatum, Blastomyces dermatitidis, Coccidioides immitis, and species of Candida.

48. The product of claim 22, wherein the tissue is normal ectopic tissue.

49. The product of claim 48, wherein said normal tissue is tissue from the ovary, thymus, parathyroid, bone marrow, or spleen.

50. The product of claims 11 or 13, wherein said subject is mammalian.

51. The product of claim 50, wherein said mammalian subject is a human or primate.

52. The product of claim 50, wherein said mammalian subject is selected from the group consisting of rodents, lagamorphs, bovines, ovines, caprines, porcines, equines, canines, felines, domestic fowl, ungulates, and bear.

53. The product of claim 11, for use in intraoperative diagnosis to identify diseased tissues.

54. The product of claim 11 for use in endoscopic diagnosis to identify diseased tissues.

55. The product of any one of claims 11 to 54, further comprising a second therapeutic agent for administration before, concurrently or after the prescribed diagnosis or treatment.

56. The product of claim 55, wherein the second therapeutic agent is a drug, naked antibody, immunomodulator, or antibody conjugate bearing a drug, radioisotope, immunomodulator or toxin.

57. A kit useful for treating or identifying diseased tissues in a subject comprising:
(A) a mutant bispecific antibody of any of claims 1 to 10;
(B) optionally, a clearing composition; and
(C) a targetable construct comprising a bivalent hapten, wherein both hapten moieties bind to the two scFvs on a single molecule of the mutant bispecific antibody, wherein the targetable construct further comprises a therapeutic cation, and/or one or more chelated or chemically bound therapeutic agents; and optionally
(D) a prodrug as defined in claim 13

58. The product of claim 26, wherein said immunomodulator is selected from the group consisting of a cytokine, a stem cell growth factor, a lymphotoxin, a hematopoietic factor, a colony stimulating factor (CSF), an interferon (IFN), erythropoietin, thrombopoietin and a combination thereof.

59. The product of claim 58, wherein said lymphotoxin is tumor necrosis factor (TNF).

60. The product of claim 58, wherein hematopoietic factor is interleukin (IL).

61. The product of claim 58, wherein said colony stimulating factor is granulocytecolony stimulating factor (G-CSF) or granulocyte macrophage-colony stimulating factor (GM-CSF).

62. The product of claim 58, wherein said interferon is interferon-α, -ß or -γ.

63. The product of claim 58, wherein said stem cell growth factor is S1 factor.

64. The product of claim 26, wherein said immunomodulator is IL-1, IL-2, IL-3, IL-6, IL-10, IL-12, IL-18, interferon-γ, TNF-α or a combination thereof.

65. The product of claim 37, wherein said neurological disease is Alzheimer's Disease.

66. The use of a mutant bispecific antibody of any of claims 1 to 10 in the preparation of an agent for treatment or diagnosis.

## Patentansprüche

1. Mutierter bi-spezifischer Antikörper, umfassend wenigstens:
(a) eine humane Gelenk-Fc-konstante Region von IgG;
(b) zwei scFvs, von denen eine jede an eine Bindungsstelle auf einem bivalenten targetierbaren Konstrukt spezifisch bindet, so dass eine eins-zu-eins Bindungswechselwirkung zwischen dem mutierten bi-spezifischen Antikörper und dem targetierbaren Konstrukt erreicht wird; und
(c) zwei Fvs,
wobei die konstante Region eine oder mehrere Aminosäuremutationen in der C_{H}2-Domäne enthält, wobei die Gelenk-konstante Region eine Mutation enthält, bei der Isoleucin an Position 253 durch Alanin oder eine Aminosäure, die von Leucin verschieden ist, ersetzt ist und wobei der Aminosäureaustausch die Klärung aus dem Blut im gleichen oder größeren Umfang verstärkt als der Austausch durch Alanin.

2. Mutierter bi-spezifischer Antikörper nach Anspruch 1, wobei die scFvs und die Fvs CDR-aufgepfropfte murine scFvs und Fvs sind.

3. Mutierter bi-spezifischer Antikörper nach Anspruch 1, wobei die scFvs und die Fvs humanisiert oder human sind.

4. Mutierter bi-spezifischer Antikörper nach Anspruch 1, umfassend die Fv-Regionen von hMN14-IgG und 734scFv.

5. Mutierter bi-spezifischer Antikörper nach Anspruch 1, wobei das bivalente targetierbare Konstrukt mit einem oder mehreren Wirkstoffen, Toxinen, Radioisotopen, Enzymen oder Markierungen konjugiert ist.

6. Mutierter bi-spezifischer Antikörper nach Anspruch 1, wobei die Fvs an ein Epitop auf einer Zielzelle binden.

7. Mutierter bi-spezifischer Antikörper nach Anspruch 6, wobei die Fvs an ein Tumorassoziiertes Antigen binden.

8. Mutierter bi-spezifischer Antikörper nach Anspruch 7, wobei die Fvs an CEA (carcinoembryonales Antigen) binden.

9. Mutierter bi-spezifischer Antikörper nach Anspruch 1, wobei der mutierte bi-spezifische Antikörper das Fv eines Klasse III anti-CEA Antikörpers enthält.

10. Mutierter bi-spezifischer Antikörper nach Anspruch 1, wobei der mutierte bi-spezifische Antikörper das scFv von Mab 679 enthält.

11. Produkt umfassend:
(A) einen mutierten bi-spezifischen Antikörper nach einem der Ansprüche 1 bis 10;
(B) optional, eine klärende Zusammensetzung; und
(C) ein targetierbares Konstrukt umfassend ein bivalentes Hapten, wobei beide Hapten-Teile an die zwei scFvs auf einem einzelnen Molekül des mutierten bi-spezifischen Antikörpers binden, wobei das targetierbare Konstrukt weiter ein therapeutisches Kation und/oder ein oder mehrere chelatgebundene oder chemisch gebundene therapeutische Agenzien umfasst; als ein Kombinationspräparat zur gleichzeitigen, getrennten oder aufeinanderfolgenden Verwendung zur Behandlung oder Diagnose einer Erkrankung eines Lebewesens.

12. Produkt nach Anspruch 11, weiter umfassend wenigstens ein zum Behandeln der Erkrankung verwendetes therapeutisches Agens.

13. Produkt nach Anspruch 11, wobei das targetierbare Konstrukt ein Enzym umfasst und das weiter umfasst:
a) einen Wirkstoffvorläufer, wobei das Enzym in der Lage ist, den Wirkstoffvorläufer an der Zielstelle in einen Wirkstoff umzuwandeln; oder
b) einen Wirkstoff, der in der Lage ist, in dem Lebewesen entgiftet zu werden, um ein Zwischenprodukt geringerer Toxizität zu bilden, wobei das Enzym in der Lage ist, das entgiftete Zwischenprodukt in eine toxische Form zurück zu verwandeln und die Toxizität des Wirkstoffs an der Zielstelle zu erhöhen; oder
c) einen Wirkstoffvorläufer, der in dem Lebewesen durch natürliche Prozesse aktiviert wird und durch Umwandlung in ein Zwischenprodukt geringerer Toxizität entgiftet wird, wobei das Enzym in der Lage ist, das entgiftete Zwischenprodukt in eine toxische Form zurück zu verwandeln, und die Toxizität des Wirkstoffes an der Zielstelle zu erhöhen.

14. Produkt nach Anspruch 13, wobei der Wirkstoffvorläufer ausgewählt ist aus der Gruppe bestehend aus Epirubicinglucuronid, CPT-11, Etoposidglucuronid, Daunomicinglucuronid und Doxorubicinglucuronid.

15. Produkt nach Anspruch 11, wobei das therapeutisches Agens ausgewählt ist aus der Gruppe bestehend aus ¹⁸F, ⁵²Fe, ⁶²Cu, ⁶⁴Cu, ⁶⁷Cu, ⁶⁷Ga, ⁶⁸Ga, ⁸⁶Y, ⁸⁹Zr, ^{94m}Tc, ⁹⁴Tc, ^{99m}Tc, ¹¹¹In, ¹²³I, ¹²⁴I, ¹²⁵I, ¹³¹I, ¹⁵⁴⁻¹⁵⁸Gd, ¹⁷⁷Lu, ³²P, ¹⁸⁸Re und ⁹⁰Y oder einer Kombination davon.

16. Produkt nach Anspruch 15, wobei das radioaktive Isotop verwendet ist, um Positronen-Emissions-Tomographie (PET) durchzuführen.

17. Produkt nach Anspruch 11, wobei das targetierbare Konstrukt ein oder mehrere Bildverstärkungsmittel zur Verwendung in der Magnet-Resonanz-Tomographie (MRI) umfasst.

18. Produkt nach Anspruch 17, wobei das Verstärkungsmittel ausgewählt ist aus der Gruppe bestehend aus Mn, Fe und Gd.

19. Produkt nach Anspruch 11, wobei das targetierbare Konstrukt ein oder mehrere Bildverstärkungsmittel zur Verwendung in der Ultraschall-Bildgebung umfasst.

20. Produkt nach Anspruch 19, wobei das targetierbare Konstrukt ein Liposom mit einem bivalenten DTPA-Peptid ist, das auf der äußeren Oberfläche der Liposomenlipidmembran kovalent angebracht ist.

21. Produkt nach Anspruch 20, wobei das Liposom gasgefüllt ist.

22. Produkt nach Anspruch 11, wobei das targetierbare Konstrukt ein oder mehrere radioaktive Isotope umfasst, die geeignet sind, um erkranktes Gewebe abzutöten.

23. Produkt nach Anspruch 22, wobei der Energiebereich des radioaktiven Isotops 60 bis 700 keV beträgt.

24. Produkt nach Anspruch 22, wobei das radioaktive Isotop ausgewählt ist aus der Gruppe bestehend aus ³²P, ³³P, ⁴⁷Sc, ⁶⁴Cu, ⁶⁷Cu, ⁶⁷Ga, ⁹⁰Y, ¹¹¹Ag, ¹¹¹In, ¹²⁵I, ¹³¹I, ¹⁴²Pr, ¹⁵³Sm, ¹⁶¹Tb, ¹⁶⁶Dy, ¹⁶⁶Ho, ¹⁷⁷Lu, ¹⁸⁶Re, ¹⁸⁸Re, ¹⁸⁹Re, ²¹²Pb, ²¹²Bi, ²¹³Bi, ²¹¹At, ²²³Ra, ²²⁵Ac und einer Kombination daraus.

25. Produkt nach Anspruch 22, wobei das targetierbare Konstrukt ¹⁰B-Atome umfasst und die Behandlung einer Erkrankung weiter den Schritt des Bestrahlens der Boratome umfasst, die an dem erkrankten Gewebe lokalisiert sind, wodurch eine BNCT des erkrankten Gewebes bewirkt wird.

26. Produkt nach Anspruch 11, wobei das therapeutische Agens ein Wirkstoff, ein Toxin, ein Hormon, ein Enzym, ein Immunmodulator, ein Chelator, eine Borverbindung, ein photoaktives Agens, ein Farbstoff oder ein Radioisotop ist.

27. Produkt nach Anspruch 11, wobei das targetierbare Konstrukt ein oder mehrere Toxine umfasst.

28. Produkt nach Anspruch 27, wobei das Toxin ausgewählt ist aus der Gruppe bestehend aus Ricin, Abrin, Ribonuklease, DNase I, Staphylokokken-Enterotoxin-A, antivirales Pokeweed-Protein, Gelonin, Diphtherietoxin, Pseudomonas-Exotoxin und Pseudomonas-Endotoxin und einer Kombination davon.

29. Produkt nach Anspruch 11, wobei das targetierbare Konstrukt ein oder mehrere Wirkstoffe umfasst.

30. Produkt nach Anspruch 29, wobei der Wirkstoff ausgewählt ist aus der Gruppe bestehend aus Stickstoffsenfgasen, Ethyleniminderivaten, Alkylsulfonaten, Nitrosoharnstoffen, Triazenen, Folsäureanalogen, Anthrazyklinen, Taxanen, COX-2-Inhibitoren, Pyrimidinanalogen, Purinanalogen, Antibiotika, Enzymen, Epipodophyllotoxinen, Platinkoordinationskomplexen, Vincaalkaloiden, substituierten Harnstoffen, Methylhydrazinderivaten, adrenocorticalen Suppressoren, Antagonisten, Endostatin, Taxolen, Camptothecinen, Doxorubicinen und deren Analogen und eine Kombination davon.

31. Produkt nach Anspruch 11, wobei das Konstrukt ein oder mehrere Agenzien zur photodynamischen Therapie umfasst.

32. Produkt nach Anspruch 31, wobei das Agens zur photodynamischen Therapie ein Photosensibilisator ist.

33. Produkt nach Anspruch 32, wobei der Photosensibilsator ausgewählt ist aus der Gruppe bestehend aus Benzoporphyrin-Monosäure-Ring A (BPD-MA), Zinn-Etiopurpurin (SnET2), sulfoniertes Aluminium-Phthalocyanin (AlSPc) und Lutetium-Texaphyrin (Lutex).

34. Produkt nach Anspruch 11, umfassend den mutierten bi-spezifischen Antikörper nach Anspruch 8, wobei die Fvs an eine Marker-Substanz binden, die mit einem Tumor assoziiert ist, und wobei der Tumor produziert oder assoziiert ist mit Antigenen, die ausgewählt sind aus der Gruppe bestehend aus Colon-spezifischem Antigen-p (CSAp), carcinoembryonalem Antigen (CEA), CD4, CD5, CD8, CD14, CD15, CD19, CD20, CD21, CD22, CD23, CD25, CD33, CD37, CD38, CD40, CD40L, CD46, CD52, CD54, CD66a-e, CD74, CD75, CD80, CD126, B7, HLA-DR, Ia, Ii, HM1.24, MUC 1, MUC2, MUC3, MUC4,Tag-72, PSMA, EGP-1, EGP-2, PSA, AFP, HCG, HCG-beta, PLAP, PAP, Histon, Tenascin, VEGF, P1GF, S100, EGFR, Insulin-ähnlichem Wachstumsfaktor, HER2/neu, organotropen Hormonen, onkogenen Produkten und Cytokeratin.

35. Produkt nach Ansprüchen 11 oder 13, wobei der mutierte bi-spezifische Antikörper das Fv eines Klasse III anti-CEA Antikörpers enthält.

36. Produkt nach Ansprüchen 11 oder 13, wobei der mutierte bi-spezifische Antikörper das scFv von Mab 679 enthält.

37. Produkt nach Anspruch 11, wobei die Erkrankung eine Immundysregulationserkrankung, eine Autoimmunkrankheit, Organ-Transplantat-Abstoßung, kardiovaskuläre Erkrankung, neurologische Erkrankung oder Graft-versus-Host-Krankheit ist.

38. Produkt nach Anspruch 37, wobei die Autoimmunkrankheit ausgewählt ist aus der Gruppe bestehend aus akuter idiopathischer thrombozytopenischer Purpura, chronischer idiopathischer thrombozytopenischer Purpura, Dermatomyositis, Sydenham-Chorea, Myasthenia gravis, systemischem Lupus erythematodes, Lupus nephritis, rheumatischem Fieber, polyglandulären Syndromen, bullösem Pemphigoid, Diabetes mellitus, Purpura Schönlein-Henoch, Poststreptokokken-Nephritis, Erythema nodosum, Takayasu-Syndrom, Addison-Krankheit, rheumatoider Arthritis, multipler Sklerose, Sarkoidose, Colitis ulcerosa, Erythema multiforme, IgA-Nephropathie, Polyarteriitis nodosa, Spondylarthritis, Goodpasture-Syndrom, Thromboangitis ubiterans, Sjögren Syndrom, primärer biliärer Zirrhose, Hashimoto-Thyreoiditis, Thyreotoxikose, Sklerodermie, chronischer aktiver Hepatitis, Polymyositis/Dermatomyositis, Polychondritis, Pemphigus vulgaris, Wegener-Granulomatose, membranöser Nephropathie, amyotropher Lateralsklerose, Tabes dorsalis, Riesenzellarteriitis/Polymyalgie, perniziöser Anämie, rapid progressivem nephritischem Syndrom und fibrosierender Alveolitis.

39. Produkt nach Ansprüchen 11 oder 13, wobei die Erkrankung durch einen Pilz, ein Virus, einen Parasiten oder ein Bakterium verursacht wird und das Fv des mutierten bi-spezifischen Antikörpers den Pilz, das Virus, den Parasiten oder das Bakterium targetiert.

40. Produkt nach Anspruch 39, wobei das Virus ausgewählt ist aus der Gruppe bestehend aus humanem Immundefizienz-Virus (HIV), Herpesvirus, Zytomegalievirus, Tollwutvirus, Influenzavirus, Hepatitis B-Virus, Sendai-Virus, felinem Leukämievirus, Reo-Virus, PolioVirus, Humanserum-Parvo-artigem Virus, Simian-Virus 40, Respiratory-Syncytial-Virus, Maus-Brusttumor-Virus, Varicella-Zoster-Virus, Dengue-Virus, Röteln-Virus, Masern-Virus, Adenovirus, humanen T-Zell-Leukämie-Viren, Epstein-Barr-Virus, murinem LeukämieVirus, Mumps-Virus, vesikulärem Stomatitis-Virus, Sindbis-Virus, lymphozytärem Choriomeningitis-Virus, Warzen-Virus und Blauzungenvirus.

41. Produkt nach Anspruch 39, wobei das Bakterium ausgewählt ist aus der Gruppe bestehend aus Anthrax bacillus, Streptococcus agalactiae, Legionella pneumophilia, Streptococcus pyogenes, Escherichia coli, Neisseria gonorrhoeae, Neisseria meningitidis, Pneumococcus, Hemophilis influenzae B, Treponema pallidum, Lyme-Krankheit-Spirochäten, Pseudomonas aeruginosa, Mycobaeterium leprae, Brucella abortus, Mycobacterium tuberculosis und Tetanustoxin.

42. Produkt nach Anspruch 39, wobei das Pathogen ein Protozoe ist.

43. Produkt nach Anspruch 42, wobei der Protozoe ausgewählt ist aus der Gruppe bestehend aus Plasmodium falciparum, Plasmodium vivax, Toxoplasma gondii, Trypanosoma rangeli, Trypanosoma cruzi, Trypanosoma rhodesiensei, Trypanosoma brucei, Schistosoma mansoni, Schistosoma japanicum, Babesia bovis, Elmeria tenella, Onchocerca volvulus, Leishmania tropica, Trichinella spiralis, Onchocerca volvulus, Theileria parva, Taenia hydatigena, Taenia ovis, Taenia saginata, Echinococcus granulosus und Mesocestoides corti.

44. Produkt nach Anspruch 39, wobei der Parasit ein Helminth oder ein Malaria-Parasit ist.

45. Produkt nach Anspruch 39, wobei das Bakterium Mycoplasma ist.

46. Produkt nach Anspruch 45, wobei das Mycoplasma ausgewählt ist aus der Gruppe bestehend aus Mycoplasma arthritidis, M. hyorhinis, M. orale, M. arginini, Acholeplasma laidlawii, M. salivarum und M. pneumoniae.

47. Produkt nach Anspruch 39, wobei der Pilz ausgewählt ist aus der Gruppe bestehend aus Histoplasma capsulatum, Blastomyces dermatitidis, Coccidioides immitis und Candida-Arten.

48. Produkt nach Anspruch 22, wobei das Gewebe normales ektopisches Gewebe ist.

49. Produkt nach Anspruch 48, wobei das normale Gewebe Gewebe aus dem Eierstock, dem Thymus, der Nebenschilddrüse, dem Knochenmark oder der Milz ist.

50. Das Produkt nach Ansprüchen 11 oder 13, wobei das Lebewesen ein Säugetier ist.

51. Produkt nach Anspruch 50, wobei das Säugetierlebewesen ein Mensch oder Primat ist.

52. Produkt nach Anspruch 50, wobei das Säugetierlebewesen ausgewählt ist aus der Gruppe bestehend aus Nagetieren, Hasentiere, Bovinae, Schafen, Ziegenartigen, Schweinen, Equinen, Caninen, Felinen, Haus-Geflügel, Huftieren und Bär.

53. Produkt nach Anspruch 11, zur Verwendung bei der intraoperativen Diagnose, um erkrankte Gewebe zu identifizieren.

54. Produkt nach Anspruch 11 zur Verwendung bei der endoskopischen Diagnose, um erkrankte Gewebe zu identifizieren.

55. Produkt nach einem der Ansprüche 11 bis 54, weiter umfassend ein zweites therapeutisches Agens zur Verabreichung vor, gleichzeitig mit oder nach der verschriebenen Diagnose oder Behandlung.

56. Produkt nach Anspruch 55, wobei das zweite therapeutische Agens ein Wirkstoff, nackter Antikörper, Immunmodulator oder Antikörperkonjugat ist, das/der einen Wirkstoff, ein Radioisotop, einen Immunmodulator oder ein Toxin trägt.

57. Kit geeignet zum Behandeln oder Identifizieren von erkrankten Geweben in einem Lebewesen, umfassend:
(A) einen mutierten bi-spezifischen Antikörper nach einem der Ansprüche 1 bis 10;
(B) optional, eine klärende Zusammensetzung; und
(C) ein targetierbares Konstrukt umfassend ein bivalentes Hapten, wobei beide Haptenteile an die zwei scFvs auf einem einzigen Molekül des mutierten bi-spezifischen Antikörpers binden, wobei das targetierbare Konstrukt weiter ein therapeutisches Kation und/oder ein oder mehrere chelatgebundene oder chemisch gebundene therapeutische Agenzien umfasst; und optional
(D) einen Wirkstoffvorläufer wie in Anspruch 13 definiert.

58. Produkt nach Anspruch 26, wobei der Immunmodulator ausgewählt ist aus der Gruppe bestehend aus einem Zytokin, einem Stammzell-Wachstumsfaktor, einem Lymphotoxin, einem hämatopoietischen Faktor, einem Kolonie-stimulierenden Faktor (CSF), einem Interferon (IFN), Erythropoietin, Thrombopoietin und einer Kombination davon.

59. Produkt nach Anspruch 58, wobei das Lymphotoxin Tumornekrosefaktor (TNF) ist.

60. Produkt nach Anspruch 58, wobei der hämatopoietische Faktor Interleukin (IL) ist.

61. Produkt nach Anspruch 58, wobei der Kolonie-stimulierende Faktor ein Granulozyten-Kolonie-stimulierender Faktor (G-CSF) oder Granulozyten-Makrophagen-Koloniestimulierender Faktor (GM-CSF) ist.

62. Produkt nach Anspruch 58, wobei das Interferon Interferon-α, -β oder -γ ist.

63. Produkt nach Anspruch 58, wobei der Stammzell-Wachstumsfaktor S1-Faktor ist.

64. Produkt nach Anspruch 26, wobei der Immunmodulator IL-1, IL-2, IL-3, IL-6, IL-10, IL-12, IL-18, Interferon-γ, TNF-α oder eine Kombination davon ist.

65. Produkt nach Anspruch 37, wobei die neurologische Erkrankung die Alzheimer'sche Erkrankung ist.

66. Verwendung eines mutierten bi-spezifischen Antikörpers nach einem der Ansprüche 1 bis 10 bei der Herstellung eines Agens zur Behandlung oder Diagnose.

## Revendications

1. Anticorps bispécifique mutant, comprenant au moins :
(a) une région constante Fc-charnière humaine provenant d'IgG ;
(b) deux scFv qui se lient chacun spécifiquement à un site de liaison sur une construction divalente permettant un ciblage de façon telle qu'une interaction de liaison individuelle est obtenue entre l'anticorps bispécifique mutant et la construction permettant un ciblage ; et
(c) deux Fv,
dans lequel ladite région constante contient une ou plusieurs mutations d'acides aminés dans le domaine C_{H}2, dans lequel ladite région constante charnière contient une mutation dans laquelle l'isoleucine en position 253 est remplacée par l'alanine, ou un acide aminé autre que la leucine, le remplacement par l'acide aminé augmentant la clairance sanguine à une valeur supérieure ou égale à celle obtenue par remplacement par l'alanine.

2. Anticorps bispécifique mutant selon la revendication 1, dans lequel lesdits scFv et lesdits Fv sont des scFv et Fv murins à CDR greffée.

3. Anticorps bispécifique mutant selon la revendication 1, dans lequel lesdits scFv et lesdits Fv sont humanisés ou humains.

4. Anticorps bispécifique mutant selon la revendication 1, comprenant les régions Fv de hMN14-IgG et le 734scFv.

5. Anticorps bispécifique mutant selon la revendication 1, dans lequel la construction divalente permettant un ciblage est conjuguée à un ou plusieurs médicaments, toxines, radio-isotopes, enzymes ou marqueurs.

6. Anticorps bispécifique mutant selon la revendication 1, dans lequel lesdits Fv se lient à un épitope sur une cellule cible.

7. Anticorps bispécifique mutant selon la revendication 6, dans lequel lesdits Fv se lient à un antigène associé aux tumeurs.

8. Anticorps bispécifique mutant selon la revendication 7, dans lequel lesdits Fv se lient au CEA (l'antigène carcinoembryonnaire).

9. Anticorps bispécifique mutant selon la revendication 1, ledit anticorps bispécifique mutant comprenant le Fv d'un anticorps anti-CEA de classe III.

10. Anticorps bispécifique mutant selon la revendication 1, ledit anticorps bispécifique mutant comprenant le scFv de Mab 679.

11. Produit comprenant :
(A) un anticorps bispécifique mutant selon l'une quelconque des revendications 1 à 10 ;
(B) facultativement, une composition de clairance ; et
(C) une construction permettant un ciblage comprenant un haptène divalent, les deux fractions de l'haptène se liant aux deux scFv présents sur une seule molécule de l'anticorps bispécifique mutant, la construction permettant un ciblage comprenant en outre un cation thérapeutique et/ou un ou plusieurs agents thérapeutiques chélatés ou chimiquement liés ;
sous forme de préparation combinée pour utilisation simultanée, séparée ou séquentielle pour le traitement ou le diagnostic d'une maladie d'un sujet.

12. Produit selon la revendication 11, lequel comprend en outre au moins un agent thérapeutique utilisé pour traiter la maladie.

13. Produit selon la revendication 11, dans lequel ladite construction permettant un ciblage comprend une enzyme et lequel comprend en outre :
a) un promédicament, ladite enzyme pouvant convertir ledit promédicament en un médicament au niveau du site cible ; ou
b) un médicament qui peut être détoxifié chez ledit sujet pour former un intermédiaire de plus faible toxicité, ladite enzyme pouvant reconvertir ledit intermédiaire détoxifié en une forme toxique et augmenter la toxicité dudit médicament au niveau du site cible ; ou
c) un promédicament qui est activé chez ledit sujet par des processus naturels et qui est susceptible de subir une détoxification par conversion en un intermédiaire de plus faible toxicité, ladite enzyme pouvant reconvertir ledit intermédiaire détoxifié en une forme toxique et augmenter la toxicité dudit médicament au niveau du site cible.

14. Produit selon la revendication 13, dans lequel ledit promédicament est choisi dans le groupe constitué par un glucuronide de l'épirubicine, le CPT-11, un glucuronide de l'étoposide, un glucuronide de la daunomycine et un glucuronide de la doxorubicine.

15. Produit selon la revendication 11, dans lequel l'agent thérapeutique est choisi dans le groupe constitué par ¹⁸F, ⁵²Fe, ⁶²Cu, ⁶⁴Cu, ⁶⁷Cu, ⁶⁷Ga, ⁶⁸Ga, ⁸⁶Y, ⁸⁹Zr, ^{94m}Tc, ⁹⁴Tc, ^{99m}Tc, ¹¹¹In, ¹²³I, ¹²⁴I, ¹²⁵I, ¹³¹I, ¹⁵⁴⁻¹⁵⁸Gd, ¹⁷⁷Lu, ³²P, ¹⁸⁸Re et ⁹⁰Y ou une association de ceux-ci.

16. Produit selon la revendication 15, dans lequel ledit isotope radioactif est utilisé pour effectuer une tomographie par émission de positons (TEP).

17. Produit selon la revendication 11, dans lequel ladite construction permettant un ciblage comprend un ou plusieurs agents d'accentuation d'image destinés à être utilisés en imagerie par résonance magnétique (IRM).

18. Produit selon la revendication 17, dans lequel ledit agent d'accentuation est choisi dans le groupe constitué par Mn, Fe et Gd.

19. Produit selon la revendication 11, dans lequel ladite construction permettant un ciblage comprend un ou plusieurs agents d'accentuation d'image destinés à être utilisés en imagerie aux ultrasons.

20. Produit selon la revendication 19, dans lequel ladite construction permettant un ciblage est un liposome ayant un conjugué peptide-DTPA divalent attaché de façon covalente à la surface extérieure de la membrane lipidique du liposome.

21. Produit selon la revendication 20, dans lequel ledit liposome est rempli de gaz.

22. Produit selon la revendication 11, dans lequel ladite construction permettant un ciblage comprend un ou plusieurs isotopes radioactifs utiles pour détruire un tissu malade.

23. Produit selon la revendication 22, dans lequel la gamme d'énergie de l'isotope radioactif est de 60 à 700 keV.

24. Produit selon la revendication 22, dans lequel ledit isotope radioactif est choisi dans le groupe constitué par ³²P, ³³P, ⁴⁷Sc, ⁶⁴Cu, ⁶⁷Cu, ⁶⁷Ga, ⁹⁰Y, ¹¹¹Ag, ¹¹¹In, ¹²⁵I, ¹³¹I, ¹⁴²Pr, ¹⁵³Sm, ¹⁶¹Tb, ¹⁶⁶Dy, ¹⁶⁶Ho, ¹⁷⁷Lu, ¹⁸⁶Re, ¹⁸⁸Re, ¹⁸⁹Re, ²¹²Pb, ²¹²Bi, ²¹³Bi, ²¹¹At, ²²³Ra, ²²⁵Ac et une association de ceux-ci.

25. Produit selon la revendication 22, dans lequel ladite construction permettant un ciblage comprend des atomes de ¹⁰B et ledit traitement d'une maladie comprenant en outre l'étape consistant à irradier lesdits atomes de bore localisés au niveau dudit tissu malade, ce qui effectue de cette manière une thérapie par capture de neutrons par le bore (BNCT) dudit tissu malade.

26. Produit selon la revendication 11, dans lequel ledit agent thérapeutique est un médicament, une toxine, une hormone, une enzyme, un immunomodulateur, un chélateur, un composé du bore, un agent photoactif, un colorant ou un radio-isotope.

27. Produit selon la revendication 11, dans lequel ladite construction permettant un ciblage comprend une ou plusieurs toxines.

28. Produit selon la revendication 27, dans lequel ladite toxine est choisie dans le groupe constitué par la ricine, l'abrine, la ribonucléase, l'ADNase 1, l'entérotoxine staphylococcique A, la protéine antivirale de la phytolaque, la gélonine, la toxine diphtérique, l'exotoxine de *Pseudomonas,* l'endotoxine de *Pseudomonas* et une association de celles-ci.

29. Produit selon la revendication 11, dans lequel ladite construction permettant un ciblage comprend un ou plusieurs médicaments.

30. Produit selon la revendication 29, dans lequel ledit médicament est choisi dans le groupe constitué par les moutardes azotées, les dérivés d'éthylèneimine, les alkylsulfonates, les nitrosourées, les triazènes, les analogues de l'acide folique, les anthracyclines, les taxanes, les inhibiteurs de la COX-2, les analogues de la pyrimidine, les analogues de la purine, les antibiotiques, les enzymes, les épipodophyllotoxines, les complexes de coordination du platine, les alcaloïdes de la pervenche, les urées substituées, les dérivés de la méthylhydrazine, les suppresseurs corticosurrénaux, les antagonistes, l'endostatine, les taxols, les camptothécines, les doxorubicines et leurs analogues, et une association de ceux-ci.

31. Produit selon la revendication 11, dans lequel la construction permettant un ciblage comprend un ou plusieurs agents de thérapie photodynamique.

32. Produit selon la revendication 31, dans lequel ledit agent de thérapie photodynamique est un photosensibilisant.

33. Produit selon la revendication 32, dans lequel ledit photosensibilisant est choisi dans le groupe constitué par le dérivé monoacide du noyau A de la benzoporphyrine (BPD-MA), l'etiopurpurine d'étain (SnET2), la phtalocyanine d'aluminium sulfonée (AlSPc) et la texaphyrine du lutétium (Lutex).

34. Produit selon la revendication 11, comprenant un anticorps bispécifique mutant selon la revendication 8 dans lequel lesdits Fv se lient à une substance marqueur associée à une tumeur et ladite tumeur produisant ou étant associée à des antigènes choisis dans le groupe constitué par l'antigène spécifique du côlon p (CSAp), l'antigène carcinoembryonnaire (CEA), les antigènes CD4, CD5, CD8, CD14, CD15, CD19, CD20, CD21, CD22, CD23, CD25, CD33, CD37, CD38, CD40, CD40L, CD46, CD52, CD54, CD66a-e, CD74, CD75, CD80, CD126, B7, HLA-DR, Ia, Ii, HM1.24, MUC1, MUC2, MUC3, MUC4, Tag-72, PSMA, EGP-1, EGP-2, PSA, AFP, HCG, HCG-bêta, PLAP, PAP, l'histone, la ténascine, le VEGF, le P1GF, la protéine S100, l'EGFR, le facteur de croissance analogue à l'insuline, le HER2/neu, les hormones organotropes, les produits oncogènes et la cytokératine.

35. Produit selon les revendications 11 ou 13, dans lequel l'anticorps bispécifique mutant comprend le Fv d'un anticorps anti-CEA de classe III.

36. Produit selon les revendications 11 ou 13, dans lequel l'anticorps bispécifique mutant comprend le scFv de Mab 679.

37. Produit selon la revendication 11, ladite maladie étant une maladie de dérèglement immunitaire, une maladie auto-immune, un rejet de greffe d'organe, une maladie cardiovasculaire, une maladie neurologique ou une maladie du greffon contre l'hôte.

38. Produit selon la revendication 37, ladite maladie auto-immune étant choisie dans le groupe constitué par le purpura thrombopénique idiopathique aigu, le purpura thrombopénique idiopathique chronique, la dermatomyosite, la chorée de Sydenham, la myasthénie grave, le lupus érythémateux disséminé, la néphropathie lupique, la fièvre rhumatismale, les syndromes polyglandulaires, la pemphigoïde bulleuse, le diabète sucré, le purpura de Henoch-Schönlein, la néphrite post-streptococcique, l'érythème noueux, l'artérite de Takayasu, la maladie d'Addison, la polyarthrite rhumatoïde, la sclérose en plaques, la sarcoïdose, la rectocolite hémorragique, l'érythème polymorphe, la glomérulonéphrite à dépôts mésangiaux d'IgA, la polyartérite noueuse, la spondylarthrite ankylosante, le syndrome de Goodpasture, la thromboangéite oblitérante, le syndrome de Sjögren, la cirrhose biliaire primitive, la thyroïdite de Hashimoto, la thyrotoxicose, la sclérodermie, l'hépatite chronique active, la polymyosite/dermatomyosite, la polychondrite, le pemphigus vulgaire, la granulomatose de Wegener, la glomérulonéphrite à dépôts extra-membraneux, la sclérose latérale amyotrophique, le tabes dorsalis, l'artérite/la polymyalgie giganto-cellulaire, l'anémie pernicieuse, la glomérulonéphrite rapidement progressive et l'alvéolite fibrosante.

39. Produit selon les revendications 11 ou 13, ladite maladie étant provoquée par un champignon, un virus, un parasite ou une bactérie et le Fv de l'anticorps bispécifique mutant ciblant le champignon, le virus, le parasite ou la bactérie.

40. Produit selon la revendication 39, le virus étant choisi dans le groupe constitué par le virus de l'immunodéficience humaine (VIH), le virus de l'herpès, le cytomégalovirus, le virus de la rage, le virus de la grippe, le virus de l'hépatite B, le virus Sendai, le virus de la leucémie féline, un réovirus, le poliovirus, un virus analogue à un parvovirus sérique humain, le virus simien 40, le virus respiratoire syncytial, le virus de la tumeur mammaire de la souris, le virus de la varicelle et du zona, le virus de la dengue, le virus de la rubéole, le virus de la rougeole, un adénovirus, les virus de la leucémie des lymphocytes T humains, le virus d'Epstein-Barr, le virus de la leucémie murine, le virus des oreillons, le virus de la stomatite vésiculaire, le virus Sindbis, le virus de la chorioméningite lymphocytaire, le virus des verrues et le virus de maladie de fièvre catarrhale du mouton.

41. Produit selon la revendication 39, ladite bactérie étant choisie dans le groupe constitué par le bacille du charbon, *Streptococcus agalactiae, Legionella pneumophilia, Streptococcus pyogenes, Escherichia coli, Neisseria gonorrhoeae, Neisseria meningitidis, Pneumococcus, Hemophilis influenzae* B*, Treponema pallidum,* le spirochète de la maladie de Lyme, *Pseudomonas aeruginosa, Mycobacterium leprae, Brucella abortus, Mycobacterium tuberculosis* et la toxine tétanique.

42. Produit selon la revendication 39, ledit pathogène étant un protozoaire.

43. Produit selon la revendication 42, ledit protozoaire étant choisi dans le groupe constitué par *Plasmodium falciparum, Plasmodium vivax, Toxoplasma gondii, Trypanosoma rangeli, Trypanosoma cruzi, Trypanosoma rhodesiensei, Trypanosoma brucei, Schistosoma mansoni, Schistosoma japanicum, Babesia bovis, Elmeria tenella, Onchocerca volvulus, Leishmania tropica, Trichinella spiralis, Onchocerca volvulus, Theileria parva, Taenia hydatigena, Taenia ovis, Taenia saginata, Echinococcus granulosus et Mesocestoides corti.*

44. Produit selon la revendication 39, ledit parasite étant un helminthe ou un hématozoaire.

45. Produit selon la revendication 39, ladite bactérie étant un mycoplasme.

46. Produit selon la revendication 45, ledit mycoplasme étant choisi dans le groupe constitué par *Mycoplasma arthritidis, M. hyorhinis, M. orale, M. arginini, Acholeplasma laidlawii, M. salivarum et M. pneumoniae.*

47. Produit selon la revendication 39, le champignon étant choisi dans le groupe constitué par *Histoplasma capsulatum, Blastomyces dermatitidis, Coccidioides immitis* et les espèces de *Candida.*

48. Produit selon la revendication 22, le tissu étant un tissu ectopique normal.

49. Produit selon la revendication 48, ledit tissu normal étant un tissu provenant d'un ovaire, du thymus, de la parathyroïde, de la moelle osseuse ou de la rate.

50. Produit selon les revendications 11 ou 13, ledit sujet étant mammifère.

51. Produit selon la revendication 50, ledit sujet mammifère étant un humain ou un primate.

52. Produit selon la revendication 50, ledit sujet mammifère étant choisi dans le groupe constitué par les rongeurs, les lagomorphes, les bovins, les ovins, les caprinés, les porcins, les équidés, les canidés, les félins, la volaille, les ongulés et l'ours.

53. Produit selon la revendication 11, destiné à être utilisé en diagnostic peropératoire pour identifier des tissus malades.

54. Produit selon la revendication 11 destiné à être utilisé en diagnostic endoscopique pour identifier des tissus malades.

55. Produit selon l'une quelconque des revendications 11 à 54, comprenant en outre un second agent thérapeutique pour administration avant, pendant ou après le diagnostic ou traitement prescrit.

56. Produit selon la revendication 55, dans lequel le second agent thérapeutique est un médicament, un anticorps nu, un immunomodulateur ou un conjugué d'anticorps portant un médicament, un radio-isotope, un immunomodulateur ou une toxine.

57. Trousse utile pour le traitement ou l'identification de tissus malades chez un sujet comprenant :
(A) un anticorps bispécifique mutant selon l'une quelconque des revendications 1 à 10 ;
(B) facultativement, une composition de clairance ; et
(C) une construction permettant un ciblage comprenant un haptène divalent, les deux fractions de l'haptène se liant aux deux scFv présents sur une seule molécule de l'anticorps bispécifique mutant, la construction permettant un ciblage comprenant en outre un cation thérapeutique et/ou un ou plusieurs agents thérapeutiques chélatés ou chimiquement liés ; et facultativement
(D) un promédicament tel que défini dans la revendication 13.

58. Produit selon la revendication 26, dans lequel ledit immunomodulateur est choisi dans le groupe constitué par une cytokine, un facteur de croissance des cellules souches, une lymphotoxine, un facteur hématopoïétique, un facteur de stimulation des colonies (CSF), un interféron (IFN), l'érythropoïétine, la thrombopoïétine et une association de ceux-ci.

59. Produit selon la revendication 58, dans lequel ladite lymphotoxine est un facteur de nécrose tumorale (TNF).

60. Produit selon la revendication 58, dans lequel ledit facteur hématopoïétique est une interleukine (IL).

61. Produit selon la revendication 58, dans lequel ledit facteur de stimulation des colonies est le facteur de stimulation des colonies de granulocytes (G-CSF) ou le facteur de stimulation des colonies de granulocytes et de macrophages (GM-CSF).

62. Produit selon la revendication 58, dans lequel ledit interféron est l'interféron α, β ou γ.

63. Produit selon la revendication 58, dans lequel ledit facteur de croissance des cellules souches est le facteur S1.

64. Produit selon la revendication 26, dans lequel ledit immunomodulateur est l'IL-1, l'IL-2, l'IL-3, l'IL-6, l'IL-10, l'IL-12, l'IL-18, l'interféron-γ, le TNF-α ou une association de ceux-ci.

65. Produit selon la revendication 37, ladite maladie neurologique étant la maladie d'Alzheimer.

66. Utilisation d'un anticorps bispécifique mutant selon l'une quelconque des revendications 1 à 10 dans la préparation d'un agent de traitement ou de diagnostic.


### SEQUENCE LISTING

<110> IMMUNOMEDICS, INC.
<120> BISPECIFIC ANTIBODY POINT MUTATIONS FOR ENHANCING RATE OF CLEARANCE
<130> 018733-1160
<140> PCT/GB03/00871
   <141> 2003-03-03
<150> 60/361,037
   <151> 2002-03-01
<160> 19
<170> PatentIn Ver. 2.1
<210> 1
   <211> 1407
   <212> DNA
   <213> Homo sapiens
<220>
   <221> CDS
   <222> (1) .. (1404)
<400> 1
   atg gga tgg agc tgt atc atc ctc ttc ttg gta gca aca gct aca ggt    48
   Met Gly Trp Ser Cys Ile Ile Leu Phe Leu Val Ala Thr Ala Thr Gly
    1               5                  10                  15
   gtc cac tcc gag gtc caa ctg gtg gag agc ggt gga ggt gtt gtg caa    96
   Val His Ser Glu Val Gln Leu Val Glu Ser Gly Gly Gly Val Val Gln
               20                  25                  30
   cct ggc cgg tcc ctg cgc ctg tcc tgc tcc gca tct ggc ttc gat ttc   144
   Pro Gly Arg Ser Leu Arg Leu Ser Cys Ser Ala Ser Gly Phe Asp Phe
           35                  40                  45
   acc aca tat tgg atg agt tgg gtg aga cag gca cct gga aaa ggt ctt   192
   Thr Thr Tyr Trp Met Ser Trp Val Arg Gln Ala Pro Gly Lys Gly Leu
       50                  55                  60
   gag tgg att gga gaa att cat cca gat agc agt acg att aac tat gcg   240
   Glu Trp Ile Gly Glu Ile His Pro Asp Ser Ser Thr Ile Asn Tyr Ala
   65                  70                  75                  80
   ccg tct cta aag gat aga ttt aca ata tcg cga gac aac gcc aag aac   288
   Pro Ser Leu Lys Asp Arg Phe Thr Ile Ser Arg Asp Asn Ala Lys Asn
                   85                  90                  95
   aca ttg ttc ctg caa atg gac agc ctg aga ccc gaa gac acc ggg gtc   336
   Thr Leu Phe Leu Gln Met Asp Ser Leu Arg Pro Glu Asp Thr Gly Val
               100                 105                 110
   tat ttt tgt gca agc ctt tac ttc ggc ttc ccc tgg ttt gct tat tgg    384
   Tyr Phe Cys Ala Ser Leu Tyr Phe Gly Phe Pro Trp Phe Ala Tyr Trp
           115                 120                 125
   ggc caa ggg acc ccg gtc acc gtc tcc tca gcc tcc acc aag ggc cca    432
   Gly Gln Gly Thr Pro Val Thr Val Ser Ser Ala Ser Thr Lys Gly Pro
       130                 135                 140
   tcg gtc ttc ccc ctg gca ccc tcc tcc aag agc acc tct ggg ggc aca    480
   Ser Val Phe Pro Leu Ala Pro Ser Ser Lys Ser Thr Ser Gly Gly Thr
   145                 150                 155                 160
   gcg gcc ctg ggc tgc ctg gtc aag gac tac ttc ccc gaa ccg gtg acg    528
   Ala Ala Leu Gly Cys Leu Val Lys Asp Tyr Phe Pro Glu Pro Val Thr
                   165                 170                 175
   gtg tcg tgg aac tca ggc gcc ctg acc agc ggc gtg cac acc ttc ccg    576
   Val Ser Trp Asn Ser Gly Ala Leu Thr Ser Gly Val His Thr Phe Pro
               180                 185                 190
   gct gtc cta cag tcc tca gga ctc tac tcc ctc agc agc gtg gtg acc    624
   Ala Val Leu Gln Ser Ser Gly Leu Tyr Ser Leu Ser Ser Val Val Thr
           195                 200                 205
   gtg ccc tcc agc agc ttg ggc acc cag acc tac atc tgc aac gtg aat    672
   Val Pro Ser Ser Ser Leu Gly Thr Gln Thr Tyr Ile Cys Asn Val Asn
       210                 215                 220
   cac aag ccc agc aac acc aag gtg gac aag aga gtt gag ccc aaa tct    720
   His Lys Pro Ser Asn Thr Lys Val Asp Lys Arg Val Glu Pro Lys Ser
   225                 230                 235                 240
   tgt gac aaa act cac aca tgc cca ccg tgc cca gca cct gaa ctc ctg    768
   Cys Asp Lys Thr His Thr Cys Pro Pro Cys Pro Ala Pro Glu Leu Leu
                   245                 250                 255
   ggg gga ccg tca gtc ttc ctc ttc ccc cca aaa ccc aag gac acc ctc    816
   Gly Gly Pro Ser Val Phe Leu Phe Pro Pro Lys Pro Lys Asp Thr Leu
               260                 265                 270
   atg atc tcc cgg acc cct gag gtc aca tgc gtg gtg gtg gac gtg agc    864
   Met Ile Ser Arg Thr Pro Glu Val Thr Cys Val Val Val Asp Val Ser
           275                 280                 285
   cac gaa gac cct gag gtc aag ttc aac tgg tac gtg gac ggc gtg gag    912
   His Glu Asp Pro Glu Val Lys Phe Asn Trp Tyr Val Asp Gly Val Glu
       290                 295                 300
   gtg cat aat gcc aag aca aag ccg cgg gag gag cag tac aac agc acg    960
   Val His Asn Ala Lys Thr Lys Pro Arg Glu Glu Gln Tyr Asn Ser Thr
   305                 310                 315                 320
   tac cgt gtg gtc agc gtc ctc acc gtc ctg cac cag gac tgg ctg aat    1008
   Tyr Arg Val Val Ser Val Leu Thr Val Leu His Gln Asp Trp Leu Asn
                   325                 330                 335
   ggc aag gag tac aag tgc aag gtc tcc aac aaa gcc ctc cca gcc ccc   1056
   Gly Lys Glu Tyr Lys Cys Lys Val Ser Asn Lys Ala Leu Pro Ala Pro
               340                 345                 350
   atc gag aaa acc atc tcc aaa gcc aaa ggg cag ccc cga gaa cca cag   1104
   Ile Glu Lys Thr Ile Ser Lys Ala Lys Gly Gln Pro Arg Glu Pro Gln
           355                 360                 365
   gtg tac acc ctg ccc cca tcc cgg gag gag atg acc aag aac cag gtc   1152
   Val Tyr Thr Leu Pro Pro Ser Arg Glu Glu Met Thr Lys Asn Gln Val
       370                 375                 380
   agc ctg acc tgc ctg gtc aaa ggc ttc tat ccc agc gac atc gcc gtg   1200
   Ser Leu Thr Cys Leu Val Lys Gly Phe Tyr Pro Ser Asp Ile Ala Val
   385                 390                 395                 400
   gag tgg gag agc aat ggg cag ccg gag aac aac tac aag acc acg cct   1248
   Glu Trp Glu Ser Asn Gly Gln Pro Glu Asn Asn Tyr Lys Thr Thr Pro
                   405                 410                 415
   ccc gtg ctg gac tcc gac ggc tcc ttc ttc ctc tat agc aag ctc acc   1296
   Pro Val Leu Asp Ser Asp Gly Ser Phe Phe Leu Tyr Ser Lys Leu Thr
               420                 425                 430
   gtg gac aag agc agg tgg cag cag ggg aac gtc ttc tca tgc tcc gtg   1344
   Val Asp Lys Ser Arg Trp Gln Gln Gly Asn Val Phe Ser Cys Ser Val
           435                 440                 445
   atg cat gag gct ctg cac aac cac tac acg cag aag agc ctc tcc ctg   1392
   Met His Glu Ala Leu His Asn His Tyr Thr Gln Lys Ser Leu Ser Leu
       450                 455                 460
   tct ccg ggt aaa tga                                                1407
   Ser Pro Gly Lys
   465
<210> 2
   <211> 468
   <212> PRT
   <213> Homo sapiens
<400> 2
   Met Gly Trp Ser Cys Ile Ile Leu Phe Leu Val Ala Thr Ala Thr Gly
   1               5                   10                  15
   Val His Ser Glu Val Gln Leu Val Glu Ser Gly Gly Gly Val Val Gln
               20                  25                  30
   Pro Gly Arg Ser Leu Arg Leu Ser Cys Ser Ala Ser Gly Phe Asp Phe
           35                  40                  45
   Thr Thr Tyr Trp Met Ser Trp Val Arg Gln Ala Pro Gly Lys Gly Leu
       50                  55                  60
   Glu Trp Ile Gly Glu Ile His Pro Asp Ser Ser Thr Ile Asn Tyr Ala
   65                  70                  75                  80
   Pro Ser Leu Lys Asp Arg Phe Thr Ile Ser Arg Asp Asn Ala Lys Asn
                   85                  90                  95
   Thr Leu Phe Leu Gln Met Asp Ser Leu Arg Pro Glu Asp Thr Gly Val
               100                 105                 110
   Tyr Phe Cys Ala Ser Leu Tyr Phe Gly Phe Pro Trp Phe Ala Tyr Trp
           115                 120                 125
   Gly Gln Gly Thr Pro Val Thr Val Ser Ser Ala Ser Thr Lys Gly Pro
       130                 135                 140
   Ser Val Phe Pro Leu Ala Pro Ser Ser Lys Ser Thr Ser Gly Gly Thr
   145                 150                 155                 160
   Ala Ala Leu Gly Cys Leu Val Lys Asp Tyr Phe Pro Glu Pro Val Thr
                   165                 170                 175
   Val Ser Trp Asn Ser Gly Ala Leu Thr Ser Gly Val His Thr Phe Pro
               180                 185                 190
   Ala Val Leu Gln Ser Ser Gly Leu Tyr Ser Leu Ser Ser Val Val Thr
           195                 200                 205
   Val Pro Ser Ser Ser Leu Gly Thr Gln Thr Tyr Ile Cys Asn Val Asn
       210                 215                 220
   His Lys Pro Ser Asn Thr Lys Val Asp Lys Arg Val Glu Pro Lys Ser
   225                 230                 235                 240
   Cys Asp Lys Thr His Thr Cys Pro Pro Cys Pro Ala Pro Glu Leu Leu
                   245                 250                 255
   Gly Gly Pro Ser Val Phe Leu Phe Pro Pro Lys Pro Lys Asp Thr Leu
               260                 265                 270
   Met Ile Ser Arg Thr Pro Glu Val Thr Cys Val Val Val Asp Val Ser
           275                 280                 285
   His Glu Asp Pro Glu Val Lys Phe Asn Trp Tyr Val Asp Gly Val Glu
       290                 295                 300
   Val His Asn Ala Lys Thr Lys Pro Arg Glu Glu Gln Tyr Asn Ser Thr
   305                 310                 315                 320
   Tyr Arg Val Val Ser Val Leu Thr Val Leu His Gln Asp Trp Leu Asn
                   325                 330                 335
   Gly Lys Glu Tyr Lys Cys Lys Val Ser Asn Lys Ala Leu Pro Ala Pro
               340                 345                 350
   Ile Glu Lys Thr Ile Ser Lys Ala Lys Gly Gln Pro Arg Glu Pro Gln
           355                 360                 365
   Val Tyr Thr Leu Pro Pro Ser Arg Glu Glu Met Thr Lys Asn Gln Val
       370                 375                 380
   Ser Leu Thr Cys Leu Val Lys Gly Phe Tyr Pro Ser Asp Ile Ala Val
   385                 390                 395                 400
   Glu Trp Glu Ser Asn Gly Gln Pro Glu Asn Asn Tyr Lys Thr Thr Pro
                   405                 410                 415
   Pro Val Leu Asp Ser Asp Gly Ser Phe Phe Leu Tyr Ser Lys Leu Thr
               420                 425                 430
   Val Asp Lys Ser Arg Trp Gln Gln Gly Asn Val Phe Ser Cys Ser Val
           435                 440                 445
   Met His Glu Ala Leu His Asn His Tyr Thr Gln Lys Ser Leu Ser Leu
       450                 455                 460
   Ser Pro Gly Lys
   465
<210> 3
   <211> 699
   <212> DNA
   <213> Homo sapiens
<220>
   <221> CDS
   <222> (1)..(696)
<400> 3
   atg gga tgg agc tgt atc atc ctc ttc ttg gta gca aca gct aca ggt    48
   Met Gly Trp Ser Cys Ile Ile Leu Phe Leu Val Ala Thr Ala Thr Gly
    1               5                  10                  15
   gtc cac tcc gac atc cag ctg acc cag agc cca agc agc ctg agc gcc    96
   Val His Ser Asp Ile Gln Leu Thr Gln Ser Pro Ser Ser Leu Ser Ala
               20                  25                  30
   agc gtg ggt gac aga gtg acc atc acc tgt aag gcc agt cag gat gtg   144
   Ser Val Gly Asp Arg Val Thr Ile Thr Cys Lys Ala Ser Gln Asp Val
           35                  40                  45
   ggt act tct gta gcc tgg tac cag cag aag cca ggt aag gct cca aag   192
   Gly Thr Ser Val Ala Trp Tyr Gln Gln Lys Pro Gly Lys Ala Pro Lys
       50                  55                  60
   ctg ctg atc tac tgg aca tcc acc cgg cac act ggt gtg cca agc aga   240
   Leu Leu Ile Tyr Trp Thr Ser Thr Arg His Thr Gly Val Pro Ser Arg
   65                  70                  75                  80
   ttc agc ggt agc ggt agc ggt acc gac ttc acc ttc acc atc agc agc   288
   Phe Ser Gly Ser Gly Ser Gly Thr Asp Phe Thr Phe Thr Ile Ser Ser
                   85                  90                  95
   ctc cag cca gag gac atc gcc acc tac tac tgc cag caa tat agc ctc   336
   Leu Gln Pro Glu Asp Ile Ala Thr Tyr Tyr Cys Gln Gln Tyr Ser Leu
               100                 105                 110
   tat cgg tcg ttc ggc caa ggg acc aag gtg gaa atc aaa cga act gtg    384
   Tyr Arg Ser Phe Gly Gln Gly Thr Lys Val Glu Ile Lys Arg Thr Val
           115                 120                 125
   gct gca cca tct gtc ttc atc ttc ccg cca tct gat gag cag ttg aaa    432
   Ala Ala Pro Ser Val Phe Ile Phe Pro Pro Ser Asp Glu Gln Leu Lys
       130                 135                 140
   tct gga act gcc tct gtt gtg tgc ctg ctg aat aac ttc tat ccc aga    480
   Ser Gly Thr Ala Ser Val Val Cys Leu Leu Asn Asn Phe Tyr Pro Arg
   145                 150                 155                 160
   gag gcc aaa gta cag tgg aag gtg gat aac gcc ctc caa tcg ggt aac    528
   Glu Ala Lys Val Gln Trp Lys Val Asp Asn Ala Leu Gln Ser Gly Asn
                   165                 170                 175
   tcc cag gag agt gtc aca gag cag gac agc aag gac agc acc tac agc    576
   Ser Gln Glu Ser Val Thr Glu Gln Asp Ser Lys Asp Ser Thr Tyr Ser
               180                 185                 190
   ctc agc agc acc ctg acg ctg agc aaa gca gac tac gag aaa cac aaa    624
   Leu Ser Ser Thr Leu Thr Leu Ser Lys Ala Asp Tyr Glu Lys His Lys
           195                 200                 205
   gtc tac gcc tgc gaa gtc acc cat cag ggc ctg agc tcg ccc gtc aca    672
   Val Tyr Ala Cys Glu Val Thr His Gln Gly Leu Ser Ser Pro Val Thr
       210                 215                 220
   aag agc ttc aac agg gga gag tgt tag                                699
   Lys Ser Phe Asn Arg Gly Glu Cys
   225                 230
<210> 4
   <211> 232
   <212> PRT
   <213> Homo sapiens
<400> 4
   Met Gly Trp Ser Cys Ile Ile Leu Phe Leu Val Ala Thr Ala Thr Gly
   1               5                   10                  15
   Val His Ser Asp Ile Gln Leu Thr Gln Ser Pro Ser Ser Leu Ser Ala
               20                  25                  30
   Ser Val Gly Asp Arg Val Thr Ile Thr Cys Lys Ala Ser Gln Asp Val
           35                  40                  45
   Gly Thr Ser Val Ala Trp Tyr Gln Gln Lys Pro Gly Lys Ala Pro Lys
       50                  55                  60
   Leu Leu Ile Tyr Trp Thr Ser Thr Arg His Thr Gly Val Pro Ser Arg
   65                  70                  75                  80
   Phe Ser Gly Ser Gly Ser Gly Thr Asp Phe Thr Phe Thr Ile Ser Ser
                   85                  90                  95
   Leu Gln Pro Glu Asp Ile Ala Thr Tyr Tyr Cys Gln Gln Tyr Ser Leu
   100 105 110
   Tyr Arg Ser Phe Gly Gln Gly Thr Lys Val Glu Ile Lys Arg Thr Val
   115 120 125
   Ala Ala Pro Ser Val Phe Ile Phe Pro Pro Ser Asp Glu Gln Leu Lys
   130 135 140
   Ser Gly Thr Ala Ser Val Val Cys Leu Leu Asn Asn Phe Tyr Pro Arg
   145 150 155 160
   Glu Ala Lys Val Gln Trp Lys Val Asp Asn Ala Leu Gln Ser Gly Asn
   165 170 175
   Ser Gln Glu Ser Val Thr Glu Gln Asp Ser Lys Asp Ser Thr Tyr Ser
   180 185 190
   Leu Ser Ser Thr Leu Thr Leu Ser Lys Ala Asp Tyr Glu Lys His Lys
   195 200 205
   Val Tyr Ala Cys Glu Val Thr His Gln Gly Leu Ser Ser Pro Val Thr
   210 215 220
   Lys Ser Phe Asn Arg Gly Glu Cys
   225 230
<210> 5
   <211> 4
   <212> PRT
   <213> Artificial Sequence
<220>
   <223> Description of Artificial Sequence: Synthetic peptide
<220>
   <223> C-term amidation
<400> 5
   Phe Lys Tyr Lys
   1
<210> 6
   <211> 4
   <212> PRT
   <213> Artificial Sequence
<220>
   <223> Description of Artificial Sequence: Synthetic peptide
<400> 6
   Phe Lys Tyr Lys
   1
<210> 7
   <211> 4
   <212> PRT
   <213> Artificial Sequence
<220>
   <223> Description of Artificial Sequence: Synthetic peptide
<220>
   <223> N-term acetylation
<220>
   <223> C-term amidation
<400> 7
   Lys Tyr Lys Lys
   1
<210> 8
   <211> 15
   <212> PRT
   <213> Artificial Sequence
<220>
   <223> Description of Artificial Sequence: Synthetic peptide linker
<400> 8
   Gly Gly Gly Gly Ser Gly Gly Gly Gly Ser Gly Gly Gly Gly Ser
   1 5 10 15
<210> 9
   <211> 4
   <212> PRT
   <213> Artificial Sequence
<220>
   <223> Description of Artificial Sequence: Synthetic peptide linker
<400> 9
   Gly Gly Gly Ser
   1
<210> 10
   <211> 56
   <212> DNA
   <213> Artificial Sequence
<220>
   <223> Description of Artificial Sequence: Primer
<400> 10
   ttctctctgc agagcccaaa tcttgtggtg gcggttcaca gctggttgtg actcag 56
<210> 11
   <211> 49
   <212> DNA
   <213> Artificial Sequence
<220>
   <223> Description of Artificial Sequence: Primer
<400> 11
   agcctccgcc tcctgatccg ccacctccta agatcttcag tttggttcc 49
<210> 12
   <211> 35
   <212> DNA
   <213> Artificial Sequence
<220>
   <223> Description of Artificial Sequence: Primer
<400> 12
   ccggaggcgg tgggagtgag gtgaaactgc aggag 35
<210> 13
   <211> 43
   <212> DNA
   <213> Artificial Sequence
<220>
   <223> Description of Artificial Sequence: Primer
<400> 13
   aaccttgagc tcggccgtcg cactcatgag gagacggtga ccg 43
<210> 14
   <211> 30
   <212> DNA
   <213> Artificial Sequence
<220>
   <223> Description of Artificial Sequence: Primer
<400> 14
   tccccgggta aaggtggcgg ttcacagctg 30
<210> 15
   <211> 17
   <212> DNA
   <213> Artificial Sequence
<220>
   <223> Description of Artificial Sequence: Primer
<400> 15
   gagctcggcc gtcgcac 17
<210> 16
   <211> 11
   <212> PRT
   <213> Homo sapiens
<400> 16
   Lys Asp Thr Leu Met Ile Ser Arg Thr Pro Glu
   1 5 10
<210> 17
   <211> 11
   <212> PRT
   <213> Homo sapiens
<400> 17
   Lys Asp Thr Leu Met Ala Ser Arg Thr Pro Glu
   1 5 10
<210> 18
   <211> 33
   <212> DNA
   <213> Artificial Sequence
<220>
   <223> Description of Artificial Sequence: Primer
<400> 18
   aaggacaccc tcatggctag ccggacccct gag 33
<210> 19
   <211> 4
   <212> PRT
   <213> Artificial Sequence
<220>
   <223> Description of Artificial Sequence: Synthetic peptide
<400> 19
   Lys Tyr Lys Lys
   1